# EUROPÄISCHE PATENTSCHRIFT

(11) **EP 1 089 823 B1**
(45) Veröffentlichungstag und Bekanntmachung des Hinweises auf die Patenterteilung: **12.11.2003**
(21) Anmeldenummer: 99929320.2
(22) Anmeldetag: 28.06.1999
(51) Int. Cl.: B03C 5/02, G01N 15/04

(54) **ELEKTRODENANORDNUNGEN ZUR ERZEUGUNG FUNKTIONELLER FELDBARRIEREN IN MIKROSYSTEMEN**
ELECTRODE ARRANGEMENT FOR GENERATING FUNCTIONAL FIELD BARRIERS IN MICROSYSTEMS
DISPOSITIF A ELECTRODES DESTINE A LA PRODUCTION DE BARRIERES DE CHAMP FONCTIONNELLES DANS DES MICROSYSTEMES

(30) Priorität: 26.06.1998 DE 19828626; 29.06.1998 DE 19828919; 20.11.1998 DE 19853658; 23.12.1998 DE 19860118
(43) Veröffentlichungstag der Anmeldung: 11.04.2001
(73) Patentinhaber: Evotec OAI AG, 22525 Hamburg (DE)
(72) Erfinder: FUHR, Günter, D-13187 Berlin (DE); SCHNELLE, Thomas, D-10243 Berlin (DE); HAGEDORN, Rolf, D-13057 Berlin (DE); MÜLLER, Torsten, D-12439 Berlin (DE)
(74) Vertreter: Hertz, Oliver, Dr.
(86) Internationale Anmeldenummer: EP9904470
(87) Internationale Veröffentlichungsnummer: WO00000293

(56) Entgegenhaltungen:
- EP-A- 0 645 169
- DE-A- 19 500 660
- US-A- 4 726 904
- US-A- 5 565 105
- FIEDLER S ET AL: "DIELECTROPHORETIC SORTING OF PARTICLES AND CELLS IN A MICROSYSTEM" ANALYTICAL CHEMISTRY, Bd. 70, Nr. 9, 1. Mai 1998 (1998-05-01), Seiten 1909-1915, XP000755524 ISSN: 0003-2700

## Beschreibung

Die Erfindung betrifft Elektrodenanordnungen zur Erzeugung funktioneller Feldbarrieren in Mikrosystemen, die zur Manipulierung suspendierter Teilchen eingerichtet sind, insbesondere funktionelle Mikroelektroden zur dielektrophoretischen Ablenkung von mikroskopischen Teilchen, und Mikrosysteme, die mit derartigen Elektrodenanordnungen ausgestattet sind sowie deren Verwendungen.

Die Manipulierung suspendierter Teilchen in fluidischen Mikrosystemen ist allgemein bekannt und wird beispielsweise von G. Fuhr et al. in "Naturwissenschaften", Bd. 81, 1994, S. 528 ff., beschrieben. Die Mikrosysteme bilden insbesondere Kanalstrukturen, die von einer Suspensionsflüssigkeit mit den zu manipulierenden Teilchen durchströmt werden. In der Regel besitzen diese Kanalstrukturen eine rechteckige Querschnittsfläche, wobei die in Betriebsposition unteren und oberen Kanalwände (Boden- und Deckflächen) eine größere Breite als die seitlichen Kanalwände (Seitenflächen) besitzen. In den Kanalstrukturen sind auf den Kanalwänden Mikroelektroden angebracht, die mit hochfrequenten elektrischen Feldern beaufschlagt werden. Unter der Wirkung der hochfrequenten elektrischen Felder werden in den suspendierten Teilchen auf der Basis negativer oder positiver Dielektrophorese Polarisationskräfte erzeugt, die eine Abstoßung von den Elektroden und in Zusammenwirkung mit Strömungskräften in der Suspensionsflüssigkeit eine Manipulierung der Teilchen im Kanal erlauben. Die Mikroelektroden herkömmlicher Mikrosysteme sind in der Regel auf den jeweils breiteren Kanalwänden als gerade Elektrodenbänder angebracht.

Zur Erzeugung der für die Dielektrophorese wirksamen hochfrequenten elektrischen Felder wirken jeweils zwei Elektrodenbänder zusammen, die an gegenüberliegenden Kanalwänden mit jeweils gleicher Gestalt und Ausrichtung angebracht sind. Die geraden Elektrodenbänder verlaufen beispielsweise parallel zur Kanalausrichtung bzw. Strömungsrichtung der Suspensionsflüssigkeit im jeweiligen Kanalabschnitt oder unter einem vorbestimmten Winkel schräg zur Kanalausrichtung. Die Elektrodenbänder besitzen zur wirksamen und sicheren Ausbildung der Polarisationskräfte an den zu manipulierenden Teilchen eine Länge, die die charakteristische Dimension der Teilchen um ein Vielfaches (Faktor rd. 20 bis 50) übersteigt.

Die herkömmlichen Mikrosysteme besitzen Nachteile in Bezug auf die Wirksamkeit der Erzeugung von Polarisationskräften, die Stabilität und Lebensdauer der Mikroelektroden und die eingeschränkte Fähigkeit, Kräftegradienten innerhalb der Kanalstruktur zu erzeugen. Diese Nachteile hängen insbesondere mit den über verhältnismäßig große Längen im Kanal gebildeten Elektrodenbändern zusammen. Je länger ein Elektrodenband ist, desto länger befindet sich ein vorbeiströmendes Teilchen im Wirkungsbereich des Elektrodenbandes, so daß auch die Wirksamkeit der jeweiligen Mikroelektrode bzw. der durch sie erzeugten Feldbarriere steigt. Andererseits sind die langen Elektrodenbänder auch störanfälliger. Durch Herstellungsfehler oder mechanische Beanspruchungen können Unterbrechungen auftreten, die zum Elektrodenausfall führen. Ferner wurden die Mikroelektroden bisher zur Erzielung einer über die Kanallänge gleichbleibenden und damit reproduzierbaren Kraftwirkung auf die genannte gerade Elektrodengestaltung beschränkt.

Aufgrund der genannten Nachteile ist auch der Einsatzbereich der genannten fluidischen Mikrosysteme mit dielektrophoretischer Teilchenmanipulierung auf die Führung der Teilchen in der Kanalstruktur oder die Ablenkung von Teilchen aus einer gegebenen Strömung beschränkt.

Die dielektrophoretische Manipulation von Partikeln in Mikrosystemen wird von S. Fiedler et. al. in "Anal. Chem." 70, 1998, S. 1909 beschrieben.

Die Aufgabe der Erfindung ist es, verbesserte Mikrosysteme zur dielektrophoretischen Teilchenablenkung zu schaffen, mit denen die Nachteile herkömmlicher Mikrosysteme überwunden werden und die insbesondere einen erweiterten Anwendungsbereich besitzen und ermöglichen, auch über kürzere Kanalabschnitte wirksame Feldbarrieren zu erzeugen. Die Aufgabe der Erfindung ist es auch, neuartige Anwendungen derartiger Mikrosysteme anzugeben.

Diese Aufgabe wird durch ein Mikrosystem mit den Merkmalen gemäß dem Patentanspruch 1 gelöst. Vorteilhafte Ausführungsformen und Anwendungen der Erfindung ergeben sich aus den abhängigen Ansprüchen.

Ein erfindungsgemäßes Mikrosystem ist insbesondere dazu eingerichtet, im Mikrosystem Feldbarrieren entlang vorbestimmter Bezugsflächen zu erzeugen, die sich zumindest teilweise über die Breite eines Kanals im Mikrosystem erstrecken und vorbestimmte Krümmungen relativ zur Längsausdehnung des Kanals, zur Strömungsrichtung der Suspensionsflüssigkeit im Kanal oder zur Bewegungsrichtung der (nicht abgelenkten) Teilchen besitzen. Mit dem Begriff "Bezugsfläche" wird in diesem Zusammenhang nicht nur ein zweidimensionales Gebilde bezeichnet, sondern ein Raumbereich, auf den sich die Feldwirkung der jeweiligen Mikroelektroden erstreckt und in dem die Feldbarriere zur dielektrischen Beeinflussung der mikroskopischen Teilchen im Mikrosystem ausgebildet ist. Dieser Raumbereich entspricht im wesentlichen einem Bereich, der von den Feldlinien der wirksamen Mikroelektroden durchsetzt wird, und erstreckt sich bei zusammenwirkenden Mikroelektrodenpaaren als gekrümmte Hyperfläche zwischen den Mikroelektroden oder bei einzeln wirkenden Mikroelektroden als Hyperfläche, die die Feldlinienverteilung der einzeln wirkenden Mikroelektrode umspannt. Die Bezugsflächen definieren die Orte, an denen Polarisationskräfte in den mikroskopischen Teilchen wirksam erzeugt werden können. Die Mikroelektroden werden so ausgebildet, daß die Bezugsflächen je nach der angestrebten Funktion der jeweiligen Mikroelektroden eine vorbestimmte Krümmung in Bezug auf die Bewegungsrichtung der Teilchen im Mikrosystem besitzen, so daß eine optimale Zusammenwirkung der Polarisationskräfte und der mechanischen Kräfte erzielt wird. Daher werden die Feldbarrieren hier auch als funktionelle Feldbarrieren bezeichnet. Der hier benutzte Begriff "Krümmung" bezieht sich nicht auf die Krümmung von Feldlinien an geraden Mikroelektroden durch das Austreten der Feldlinien in den angrenzenden Raum. Die Krümmung bezeichnet vielmehr die Gestaltung der an Mikroelektroden ausgebildeten Feldbarrieren.

Die Feldbarrieren mit den erfindungsgemäß gekrümmten Bezugsflächen werden vorzugsweise nach einer der drei folgenden Grundformen gestaltet. Gemäß einer ersten Variante besteht eine erfindungsgemäße Elektrodenanordnung aus mindestens einer bandförmigen, gekrümmten Mikroelektrode, die sich auf der breiteren Kanalwand (Boden- und/oder Deckfläche) zumindest teilweise über die Kanalbreite erstreckt. Bei einer zweiten Variante ist mindestens eine Mikroelektrode vorgesehen, die an der schmaleren Kanalwand (Seitenfläche) angebracht ist. Bei der dritten Variante sind mindestens eine Mikroelektrode auf der Boden- und/oder Deckfläche des Kanals und mindestens eine Hilfselektrode mit Abstand von der Boden- oder Seitenfläche des Kanals angebracht. Die Hilfselektrode liefert eine Deformation der von der Mikroelektrode oder den Mikroelektroden an den Boden- bzw. Seitenflächen des Kanals ausgehenden Feldlinien, so daß die erfindungsgemäß gekrümmten Bezugsflächen gebildet werden. Bei allen Varianten können die jeweiligen Elektroden (Mikroelektroden, Hilfselektroden) an sich band- oder punktförmig oder flächig ausgebildet sein. Die Elektrodenanordnungen der zweiten und dritten Variante werden auch als dreidimensionale Elektrodenanordnungen bezeichnet, da dabei Mikroelektroden eingesetzt werden, die aus den Ebenen der Boden- oder Seitenflächen des Kanals herausragen oder von diesen mit Abstand angeordnet sind.

Gegenstand der Erfindung ist somit die Optimierung von Mikroelektroden in Bezug auf ihre Wirkung auf suspendierte Teilchen, die natürliche oder synthetische Teilchen umfassen können, z.B. zur Erzeugung maximaler Kräfte bei gleichzeitig minimierten elektrischen Verlusten.

Die Erfindung besitzt die folgenden Vorteile. Die Gestaltung der Mikroelektroden kann z.B. an das Strömungsprofil in der Suspensionsflüssigkeit angepaßt werden. Dies liefert den Vorteil, daß die Mikroelektroden kürzer ausgebildet werden und zur Erzeugung geringerer Barrieren ausgelegt sein können, jedoch die gleiche Effektivität wie herkömmliche Mikroelektroden in Form gerader Bänder besitzen. Dies wirkt sich vorteilhaft auf die Lebensdauer und Funktionstüchtigkeit der Mikroelektroden und somit der gesamten Mikrosysteme aus. Außerdem kann der in einem Mikrosystem verfügbare Platz effektiver genutzt werden. Es werden ferner Elektrodenanordnungen bereitgestellt, mit denen Gradienten und somit in Abhängigkeit vom jeweiligen Kanalbereich verschieden starke Kräfte erzeugt werden können. Es ist beispielsweise vorgesehen, daß die Feldbarrieren der Mikroelektroden so gestaltet sind, daß an den Teilchen in der Mitte des Kanals größere Polarisationskräfte ausgeübt werden als am Rand des Kanals.

Die erfindungsgemäße Ausbildung von Feldbarrieren entlang gekrümmter Bezugsflächen ermöglicht auch die Schaffung neuartiger Anwendungen von Mikrosystemen, insbesondere zum Lenken von suspendierten Teilchen in bestimmte Kanalbereiche, zum Sortieren von suspendierten Teilchen nach ihren passiven elektrischen Eigenschaften oder zum Sammeln oder Haltern suspendierter Teilchen in bestimmten Kanalabschnitten. Zur letztgenannten Anwendung werden die Mikroelektroden mit einer geometrischen Ausformung zur Halterung der Teilchen in einem Lösungsstrom oder zur Erzeugung einer Teilchenformation ausgebildet. All die genannten Anwendungen liefern eine gegenüber dem Mikrosystem berührungsfreie Manipulierung der suspendierten Teilchen, was besonders wesentlich für die Manipulierung biologischer Zellen oder Zellbestandteile ist.

Bevorzugte Anwendungen liegen in der Mikrosystemtechnik zur Separation, Manipulierung, Beladung, Fusion, Permeation, Pärchenbilden und Aggregatformation von mikroskopisch kleinen Teilchen.

Die Teilchenbewegung in einem Mikrosystem mit erfindungsgemäßen Elektrodenformen kann unter der Wirkung von Zentrifugal- und/oder Gravitationskräfen erfolgen.

Weitere Einzelheiten und Vorteile der Erfindung werden aus den im folgenden beschriebenen Zeichnungen ersichtlich. Es zeigen:
- Fign. 1a bis 1d:: schematische Perspektivansichten einer Kanalstruktur mit Mikroelektroden zur Erzeugung von Feldbarrieren in einem Mikrokanal und Beispiele erfindungsgemäß gekrümmter Bezugsflächen;
- Fig. 2:: eine schematische Draufsicht auf bandförmige, gekrümmte Mikroelektroden;
- Fig. 3:: eine schematische Draufsicht auf eine abgewandelte Gestaltung bandförmiger, gekrümmter Mikroelektroden;
- Fign. 4a bis 4c:: schematische Ansichten zur Illustration von Sortierelektroden zur Teilchensortierung;
- Fign. 5a und 5b:: schematische Ansichten von Mikroelektroden zur Erzeugung von Feldgradienten;
- Fign. 6a bis 6e:: schematische Ansichten bandförmiger Fangelektroden;
- Fign. 7a bis 7c:: weitere Fangelektroden;
- Fig. 8:: eine Draufsicht auf verschiedene Elektrodenanordnungen zur Erzeugung von gekrümmten Feldbarrieren;
- Fig. 9:: eine schematische Ansicht einer Elektrodenanordnung an Seitenwänden eines Kanals;
- Fign. 10 bis 12:: verschiedene dreidimensionaler Elektrodenanordnungen;
- Fig. 13:: eine schematische Draufsicht auf eine segmentierte Elektrodenanordnung; und
- Fign. 14 bis 16: Beispiele mit einem Mikrosystem mit Zentrifugal- und/oder Gravitationsantrieb der Teilchenbe wegung.

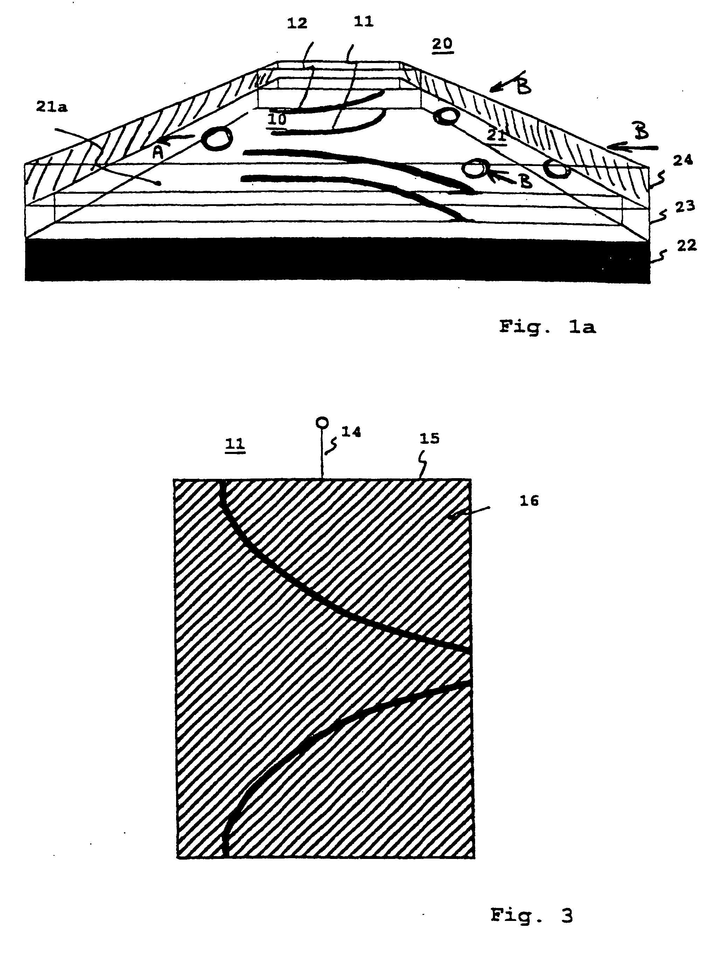

Fig. 1a zeigt in schematischer Form beispielhaft die Ausführung von Mikroelektroden zur Erzeugung von Feldbarrieren in Mikrokanälen. Das fluidische Mikrosystem 20 ist ausschnittsweise in überhöht perspektivischer Seitenansicht einer Kanalstruktur dargestellt. Der Kanal 21 wird durch zwei mit Abstand auf einem Substrat 22 angeordnete Spacer 23 gebildet, die ein Deckteil 24 tragen. Die Kanalbreite und -höhe betragen rd. 200 um bzw. 40 µm, können aber auch kleiner sein. Derartige Strukturen werden beispielsweise mit den an sich bekannten Prozessierungstechniken der Halbleitertechnologie hergestellt. Das Substrat 22 bildet die Bodenfläche 21a des Kanals 21. Dementsprechend wird die Deckfläche 21b (aus Übersichtlichkeitsgründen nicht gesondert hervorgehoben) durch das Deckteil 24 gebildet. Die Elektrodenanordnung 10 besteht aus Mikroelektroden 11, 12, die auf der Bodenfläche 21a bzw. auf der Deckfläche 21b angebracht sind. Jede der Mikroelektroden 11, 12 besteht aus gekrümmten Elektrodenbändern, die unten näher beschrieben werden.

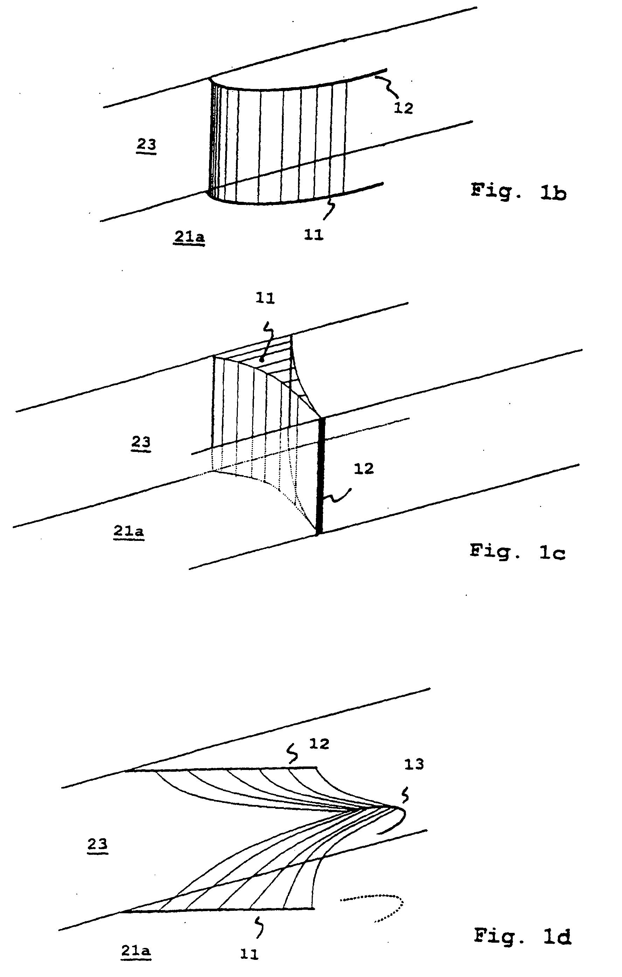

In Fig. 1a bilden die Elektrodenbänder eine Elektrodenstruktur, die im einzelnen unten unter Bezug auf die Fig. 2 erläutert wird. Die anderen, im folgenden beschriebenen Ausführungsformen erfindungsgemäßer Elektrodenanordnungen können entsprechend auf den Boden-, Deck- und/oder Seitenflächen des Kanals 21 angebracht sein. Der Mikrokanal 21 wird von einer Suspensionsflüssigkeit durchströmt (im Bild von rechts nach links), in der Partikel 30 suspendiert sind. Die in Fig. 1a dargestellte Elektrodenanordnung 10 besitzt beispielsweise die Aufgabe, die Partikel 30 von verschiedenen Bewegungsbahnen innerhalb des Kanals auf eine mittlere Bewegungsbahn gemäß Pfeil A zu führen. Hierzu werden die Mikroelektroden 11, 12 derart mit elektrischen Potentialen beaufschlagt, daß sich im Kanal elektrische Feldbarrieren ausbilden, die die von rechts anströmenden Teilchen hin zur Kanalmitte (Pfeilrichtungen B) zwingen.

Die typischen Abmessungen der Mikroelektroden 11, 12 liegen bei einer Breite von 0,1 bis zu einigen zehn Mikrometern (typischerweise 5 ... 10 µm), einer Dicke von 100 nm bis zu einigen Mikrometern (typischerweise 200 nm) und einer Länge von bis zu mehreren hundert Mikrometern. Das Innere des Kanals 21 wird durch die auf der Ober- und Unterseite der Teile 23, 24 prozessierten Elektroden auf Grund der geringfügigen Dicke der Elektroden nicht eingeschränkt. Das Teil 23 ist ein Spacer, dessen Strukturierung die seitlichen Kanalwände bildet.

Die Mikroelektroden 11, 12 werden mittels hochfrequenter elektrischer Signale (typischerweise mit einer Frequenz im MHz-Bereich und einer Amplitude im Voltbereich) angesteuert. Die jeweils gegenüberliegenden Elektroden 11a, 11b bilden ein Ansteuerpaar, wenngleich auch die in einer Ebene liegenden Elektroden in ihrer Ansteuerung (Phase, Frequenz, Amplitude) zusammenwirken können. Das durch den Kanal 21, d.h. senkrecht zur Strömungsrichtung erzeugte elektrische Hochfrequenzfeld wirkt auf suspendierte Teilchen 30 (die auch lebende Zellen oder Viren sein können) polarisierend. Bei den genannten Frequenzen und geeigneter Leitfähigkeit der die Teilchen umgebenden Suspensionsflüssigkeit werden die Teilchen von den Elektroden abgestoßen. Damit läßt sich der hydrodynamisch offene Kanal 21 über die elektrischen Felder an- und abschaltbar strukturieren, kompartimentieren bzw. lassen sich die Bewegungsbahnen der Teilchen im passiven Strömungsfeld beeinflussen. Desweiteren ist es möglich, die Teilchen trotz permanenter Strömung zu retardieren bzw. auch ortsstabil ohne Berührung einer Oberfläche zu positionieren.

Im folgenden werden Gestaltungsformen von Elektrodenanordnungen beschrieben, wobei aus Übersichtlichkeitsgründen in den Figuren 2 bis 13 ggf. nur eine planare Elektrodenanordnung (oder Teile einer solchen), z.B. auf der Bodenfläche des Kanals, dargestellt ist.

Die Fign. 1b bis 1c zeigen die Grundformen von Feldbarrieren oder elektromagnetischen Begrenzungen, die mit erfindungsgemäßen Elektrodenanordnungen gemäß den obengenannten Varianten realisiert werden. Die Illustrationen sind Prinzipdarstellungen der Bezugsflächen, auf denen die Feldbarrieren mit erfindungsgemäßen Mikroelektroden ausgebildet werden. Aus Übersichtlichkeitsgründen sind jeweils nur Teile der Seitenfläche (Spacer 23) und der Bodenfläche 21a des Kanals, die Mikroelektroden 11, 12 und der Verlauf der Bezugsflächen (schraffiert) gezeigt.

Gemäß der obengenannten ersten Variante wird die Feldbarriere im Kanal zwischen zwei gekrümmten Mikroelektroden 11, 12 auf den Boden- bzw. Deckflächen des Kanals gebildet (Fig. 1b). Die Bezugsfläche der Feldbarriere (schraffiert dargestellt) verläuft entsprechend als gekrümmte, auf den Boden- und Deckflächen senkrecht stehende Fläche. Sind die Mikroelektroden 11, 12 beispielsweise entsprechend einem bestimmten hyperbolischen Strömungsprofil gekrümmt (s. unten), so bildet die Bezugsfläche den Ausschnitt der Mantelfläche eines hyperbolischen Zylinders. Falls die Mikroelektroden 11, 12 nicht genau übereinander angeordnet sind, so wird die Bezugsfläche auch noch in Bezug auf die Boden- und Deckflächen des Kanals schiefwinklig.

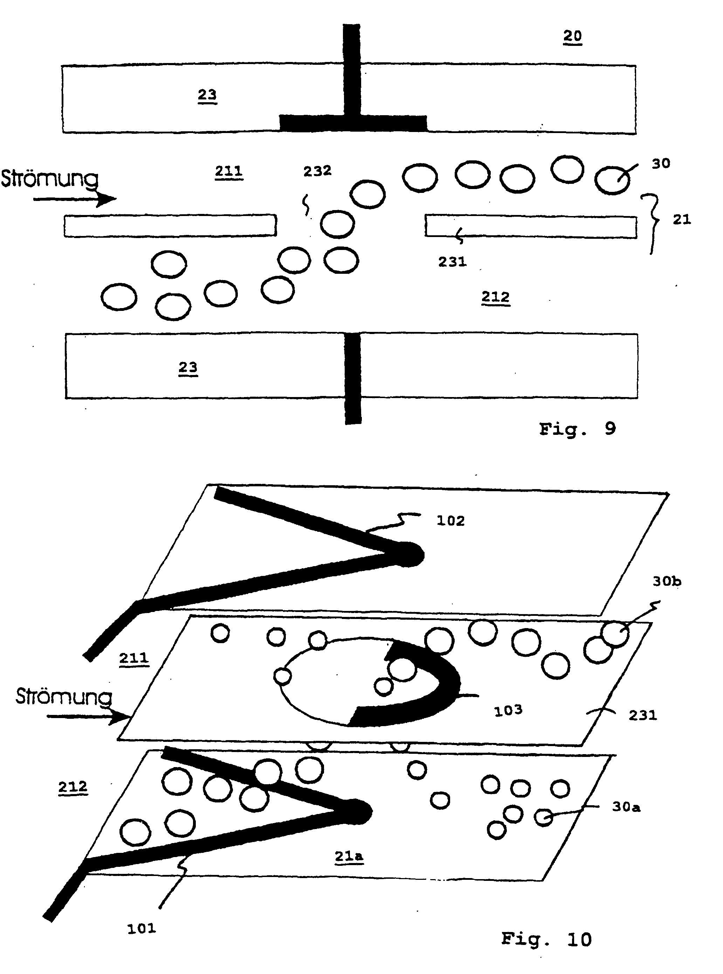

Gemäß Fig. 1c umspannt die schraffiert dargestellte Bezugsfläche einen Raumbereich, der von Feldlinien durchsetzt wird, die von einer Mikroelektrode 11 an einer Seitenfläche des Kanals zu einer Mikroelektrode 12 an der gegenüberliegenden Seitenfläche verlaufen. Beim dargestellten Beispiel besitzt die erste Mikroelektrode 11 eine größere Fläche als die zweite Mikroelektrode 12, so daß bei der letzteren eine Feldlinienkonzentration auftritt. Dadurch sind die von der Feldbarriere auf suspendierte Teilchen wirkenden Polarisationskräfte nahe der zweiten Mikroelektrode 12 größer als nahe der ersten Mikroelektrode 11 (s. auch Fig. 9).

Die obengenannte dritte Variante mit einer dreidimensionalen Elektrodenanordnung ist in Fig. 1d illustriert. Die Mikroelektroden 11, 12 befinden sich auf den Boden- bzw. Deckflächen des Kanals, während die Hilfselektrode 13 mit einer geeigneten Halterung in der Kanalmitte angeordnet ist (s. auch Fig. 10). Durch die Hilfselektrode 13 werden die Feldlinien zwischen den Mikroelektroden 11, 12 verzerrt, so daß sich die schraffiert dargestellte, gekrümmte Bezugsfläche (teilweise gezeigt) ergibt.

Die illustrierten Bezugsflächen stellen lediglich die Position der Feldbarrieren dar, ohne auch die in den entsprechenden Bereichen wirkenden Kräfte, d.h. die Höhe der Feldbarrieren, zu illustrieren. Die wirkenden Kräfte hängen im wesentlichen von der Feldliniendichte und den passiven elektrischen Eigenschaften der zu manipulierenden Teilchen im jeweiligen Kanalbereich ab. Die erfindungsgemäßen funktionellen Feldbarrieren werden somit durch die geometrische Gestalt der zusammenwirkenden Mikroelektroden sowohl in Bezug auf deren Form (Krümmungen usw.), da die dielektrophoretischen Abstoßungskräfte im wesentlichen senkrecht auf den Bezugsflächen stehen, als auch in Bezug auf deren Flächen (Feldliniendichte) beeinflußt.

Eine erfindungsgemäße Elektrodenanordnung 10 entsprechend der obengenannten ersten Variante ist in Fig. 2 dargestellt. Auf der Bodenfläche 21a des seitlich durch die Spacer 23 begrenzten Kanals eines Mikrosystems sind Mikroelektroden 11a, 11b angeordnet. Die Mikroelektroden 11a, 11b werden über die Steuerleitungen 14 mit hochfrequenten elektrischen Potentialen beaufschlagt und wirken zur Bildung eines sogenannten Partikeltrichters wie folgt zusammen.

Die Elektrodenanordnung 10 ist dazu vorgesehen, die zunächst in der gesamten Kanalbreite bzw. dem gesamten Kanalvolumen anströmenden Teilchen 30a berührungslos auf eine Mittellinie des Kanals zu fokussieren, wie dies durch die Position des Teilchens 30b illustriert ist. Der Vorteil dieser Anordnung besteht in der Optimierung der Elektrodenbänder in Bezug auf die Sicherheit der Ablenkung (Fokussierung) der suspendierten Teilchen, die Verkürzung der Elektrodenanordnung in Kanallängsrichtung und die Verringerung der elektrischen Verluste an den Mikroelektroden.

Bei dieser Ausführungsform der Erfindung besteht die Grundidee der Gestaltung der Mikroelektroden darin, die Krümmung der durch die Feldbarriere gebildeten Bezugsflächen an die Strömungskräfte im Kanal anzupassen. In Mikrosystemen mit Kanaldimensionen unterhalb von 500 µm erfolgt nämlich wegen der bei diesen Dimensionen geringen Reynolds-Zahlen die Ausbildung laminarer Strömungen mit vorbestimmten Strömungsprofilen. Die Strömungsgeschwindigkeit in der Nähe der Kanalwände ist geringer als in der Kanalmitte (Strömungsgeschwindigkeit unmittelbar an der Kanalwand gleich Null). Dadurch treten in der Nähe der Kanalwände geringere Strömungskräfte als in der Kanalmitte auf. Dies ermöglicht eine Manipulierung der Teilchen am Kanalrand mit geringeren Polarisationskräften oder mit steiler gegen die Strömungskräfte gerichteten Polarisationskräften als in der Kanalmitte. Das Zusammenwirken der Strömungs- und Polarisationskräfte wird unten erläutert. Werden entlang der gesamten Länge der Mikroelektroden im wesentlichen gleiche Polarisationskräfte ausgebildet, so genügt es für eine sichere Ablenkung, daß die zu manipulierenden Teilchen am Kanalrand auf steiler in den Kanal ragende Mikroelektroden treffen als in der Kanalmitte. Dies erlaubt eine wesentliche Verkürzung der Mikroelektroden (s. unten).

Die auf die Teilchen wirkenden Kräfte sind in Fig. 2 beispielhaft in einzelnen Abschnitten der Mikroelektrode 11a illustriert. Die jeweilige Gesamtkraft setzt sich aus der elektrisch induzierten Abstoßungskraft F_{P} (Polarisationskraft) und der Antriebskraft F_{S} zusammen, die durch die Strömung der Suspensionsflüssigkeit oder auch von außen (z.B. in Zentrifugalsystemen als Zentrifugalkraft) ausgeübt wird. Die resultierende Gesamtkraft F_{R} ergibt sich durch Vektoraddition der Kräfte F_{P} und F_{S}. Schneidet der Vektor der Gesamtkraft F_{R} die Feldbarriere der Mikroelektrode 11a nicht, so wird ein Teilchen sicher abgelenkt. Die Kräftediagramme in Fig. 2 illustrieren, daß die Antriebskraft F_{S} hin zur Kanalmitte zunimmt. Zur Erfüllung der genannten Bedingung zur sicheren Teilchenablenkung ändert sich dementsprechend der Winkel zwischen der Ausrichtung der Mikroelektrode 11a und der Kanallängsrichtung von einem steileren Winkel am Kanalrand hin zu einem geringen Winkel (nahezu Parallelität) in Kanalmitte.

Die Mikroelektroden 11a, 11b sind somit in Abhängigkeit vom Strömungsprofil gekrümmt ausgebildet. Bei der dargestellten Ausführungsform besteht jede der bandförmigen Mikroelektroden aus einer Vielzahl jeweils gerader Elektrodenabschnitte. Bei einer abgewandelten Ausführungsform kann aber auch ein stetiger Krümmungsverlauf vorgesehen sein. Der Krümmungsverlauf ist entsprechend den in laminaren Strömungen auftretenden parabeloder hyperbelförmigen Strömungsprofilen entsprechend auch parabel- oder hyperbelförmig.

Die Mikroelektroden 11a, 11b bilden erfindungsgemäß die Feldbarrieren entlang einer gekrümmten Bezugsfläche.

Die Mikroelektroden 11c, 11d sind in der Praxis nicht vorgesehen und dienen in der Darstellung dem Vergleich einer erfindungsgemäßen Anordnung von polygonal gekrümmten Mikroelektroden mit geraden Elektrodenbändern gleicher Ablenkleistung. Es zeigt sich, daß die erfindungsgemäßen Mikroelektroden 11a, 11b deutlich kürzer sind.

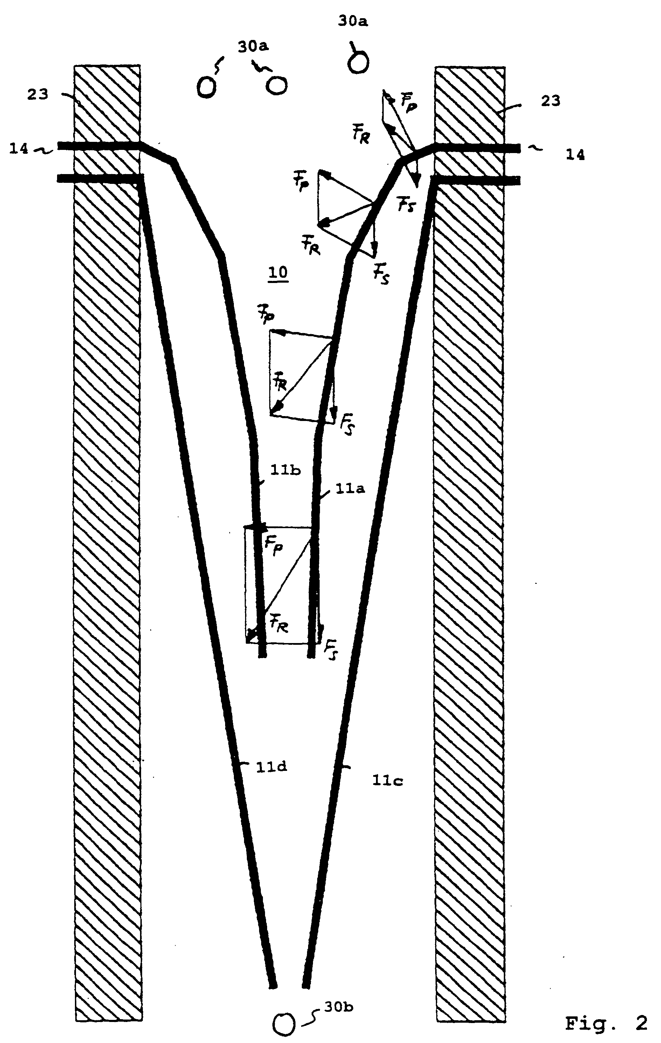

Die in Fig. 2 gezeigten schmalen Elektrodenbänder sind gegenüber Herstellungsfehlern und lokalen Unterbrechungen sehr empfindlich. Ein Haarriß am Ansatz einer bandförmigen Mikroelektrode führt zum Ausfall der gesamten Mikroelektrode. Dem kann mit einer Elektrodengestaltung abgeholfen werden, die schematisch in Fig. 3 gezeigt ist. Die zu Fig. 3 beschriebene Strukturierungs- und Abdecktechnik kann auch bei den anderen Ausführungsformen der Erfindung implementiert werden.

Fig. 3 zeigt eine Mikroelektrode 11 mit einer Steuerleitung 14. Die Elektrode 11 besteht aus einer elektrisch leitenden Schicht 15, die eine elektrisch nichtleitende Isolations- oder Deckschicht 16 trägt. Die Isolationsschicht 16 besitzt eine Strukturierung in Form von Ausnehmungen, durch die die Schicht 15 freiliegt. In Fig. 3 ist die Isolationshicht 16 schraffiert und die (z.B. metallische) Schicht 15 schwarz gezeichnet. Die Strukturierung der Isolationsschicht erfolgt entsprechend der gewünschten Form von Mikroelektroden, die im dargestellten Beispiel zur Bildung eines Partikeltrichters wie in Fig. 2 eingerichtet sind. Die elektrischen Feldlinien treten von der metallischen Schicht 15 in den Kanal nur in den Bereichen der Ausnehmungen, so daß wiederum Feldbarrieren mit anwendungsabhängig gekrümmten Bezugsflächen gebildet werden. Diese Gestaltung besitzt den Vorteil, daß eine geringfügige Unterbrechung der freiligenden Abschnitte der metallischen Schicht 15 (d.h. der Mikroelektrode) keinen Ausfall bedeutet, da über die restliche metallische Schicht 15 auch die übrigen freiligenden Bereiche der Mikroelektrode mit den jeweiligen Potentialen beaufschlagt werden. Die Schicht 15 besitzt beispielsweise eine Dicke von rd. 50 nm bis zu einigen µm, typischerweise rd. 200 nm. Die Dicke der Isolationsschicht beträgt rd. 100 nm bis zu einigen µm. Die Isolationsschicht besteht vorzugsweise aus biokompatiblen Materialien (z.B. Oxide, SiO₂, SiNO₃ und dergleichen, Polymere, Tantalverbindungen oder dergleichen).

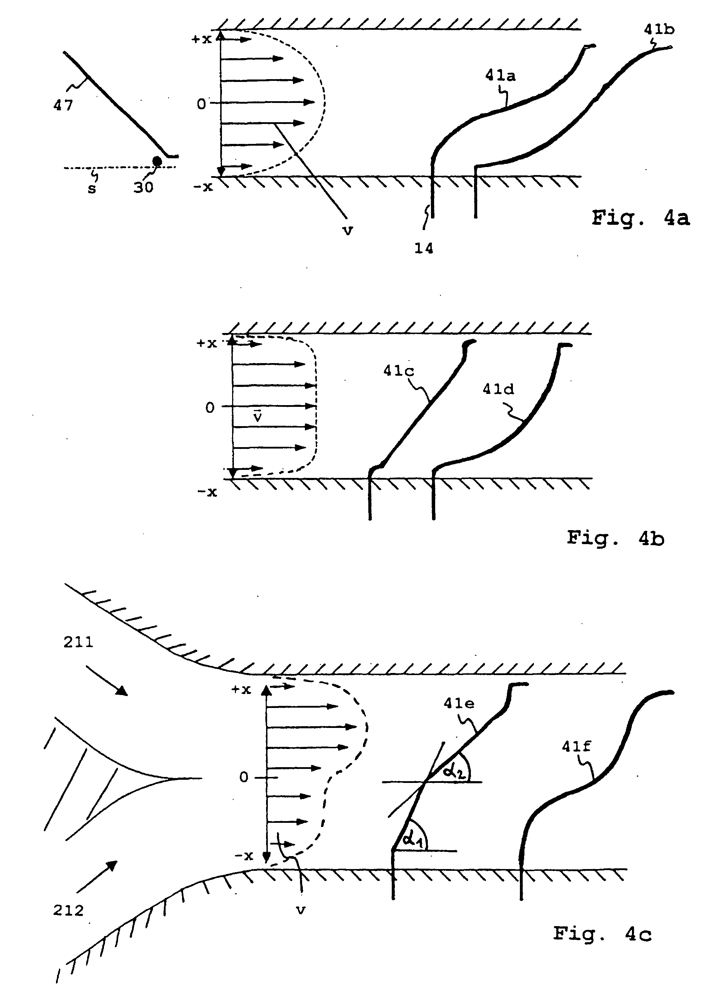

Eine weitere Ausführungsform einer erfindungsgemäßen Elektrodenanordnung 10 entsprechend der obengenannten ersten Variante wird im folgenden unter Bezug auf die Fign. 4a bis 4c erläutert. Eine wichtige Anwendung fluidischer Mikrosysteme besteht in der Sortierung der suspendierten Teilchen in Abhängigkeit von deren passiven elektrischen Eigenschaften (im folgenden auch als Polarisationseigenschaften bei negativer Dielektrophorese bezeichnet). Die Polarisationseigenschaften hängen von den dielektrischen Eigenschaften der Teilchen und deren Ausmaßen ab. Die dielektrischen Eigenschaften biologischer Zellen sind ein empfindlicher Indikator bestimmter Zelleigenschaften oder -veränderungen, die an sich etwa durch eine Größenbeobachtung nicht erfaßbar wären.

Eine Teilchensortierung in Abhängigkeit von ihren passiven elektrischen Eigenschaften basiert auf dem folgenden Prinzip. Ob ein Teilchen die von einer Sortierelektrode ausgebildete Feldbarriere passieren kann, hängt davon ab, ob die resultierende Kraft aus der Antriebskraft F_{S} und der Polarisationskraft F_{P} (s. oben) die Feldbarriere schneidet oder nicht. Weist die resultierende Gesamtkraft F_{R} durch die Feldbarriere hindurch, so bewegt sich das Teilchen in diese Richtung, d.h. die Sortierelektrode wird passiert. Weist die resultierende Kraft F_{R} jedoch in einen in Bezug auf die Sortierelektrode stromaufwärts gelegenen Bereich, so wird sich das Teilchen in diese Richtung bewegen und nicht die Sortierelektrode passieren können. Die resultierende Kraft F_{R} hängt, wie oben erläutert wurde, von der Strömungsgeschwindigkeit des Kanals und somit von der x-Position des Teilchens ab. Hin zur Kanalmitte nimmt die Strömungsgeschwindigkeit zu. Damit werden Teilchen mit relativ großer Polarisierbarkeit, die am Kanalrand die Sortierelektrode nicht passieren konnten, hin zur Kanalmitte einer stärkeren Antriebskraft Fₛ ausgesetzt, so daß dann gegebenenfalls ein Vorbeitritt an der Sortierelektrode möglich ist. Die Änderung der Strömungsgeschwindigkeit in x-Richtung folgt dem Strömungsprofil und ist in der Regel nicht-linear. Dadurch würde sich bei Einsatz einer geraden Sortierelektrode ein nichtlineares Trennverhalten ergeben. Dies wird durch die Implementierung erfindungsgemäß gekrümmter Feldbarrieren kompensiert. Hierzu werden Mikroelektroden 41a, 41b mit einer Krümmung in Abhängigkeit vom Strömungsprofil nach den unter Bezug auf Fig. 2 erläuterten Prinzipien eingesetzt.

Fig. 4a zeigt zwei Beispiele gekrümmter Mikroelektroden 41a, 41b auf der Bodenfläche 21a eines Kanals zwischen seitlichen Spacern 23. Der Kanal wird in y-Richtung von links nach rechts durchströmt, wobei die Pfeile v das Geschwindigkeits-Strömungsprofil im Kanal darstellen. Stromaufwärts vor der eigentlichen Sortierelektrode 41a oder 41b befindet sich eine geradlinige Mikroelektrode 47, deren Aufgabe darin besteht, die von links anströmenden Teilchen 30 auf eine Startlinie s zu fokussieren. Die Mikroelektrode 47 kann auch als Fokussierelektrode bezeichnet werden. Sie ist (wie dargestellt) als gerade, herkömmliche Ablenkelektrode oder auch gekrümmt ausgeführt. Stromabwärts von der Fokussierelektrode 47 ist eine der Sortierelektroden 41a oder 41b angeordnet, deren Aufgabe darin besteht, die anströmenden Teilchen 30 in Abhängigkeit von ihren Polarisationseigenschaften in bezüglich der x-Richtung verschiedene Bahnen im Kanal zu überführen. Die Teilchen mit einer hohen Polarisierbarkeit 30a sollen sich von den Teilchen mit einer geringen Polarisierbarkeit 30b in y-Richtung auf verschiedenen Bahnen weiter bewegen.

Die Sortierelektrode 41a ist für eine lineare Kraftwirkung eingerichtet. Hierzu ist die Krümmung der Mikroelektrode entsprechend dem Strömungsprofil ausgebildet. Bei geringen Strömungsgeschwindigkeiten ist ein starker Anstellwinkel zwischen der Mikroelektrode und der y-Richtung und bei größeren Strömungsgeschwindigkeiten ein geringerer Anstellwinkel ausgebildet. Die Mikroelektrode 41a besitzt somit eine S-Form mit einem Wendepunkt in Kanalmitte. Nach Passage der Sortierelektrode 41a besteht ein linearer Zusammenhang zwischen der x-Koordinate des Teilchens und seiner Polarisierbarkeit. Ist eine nichtlineare Sortierwirkung beabsichtigt, so kann die Mikroelektrode wie die Sortierelektrode 11b gekrümmt sein. Die Krümmung ist schwächer als im Falle der Sortierelektrode 11a, so daß der Einfluß der Antriebskraft Fₛ durch die Strömungsgeschwindigkeit nicht kompensiert wird. Je nach den eingestellten Verhältnissen ergibt sich ein nichtlinearer Zusammenhang zwischen der x-Position der Teilchen und ihrer Polarisierbarkeit nach Passage der Sortierelektrode 11b. Diese Gestaltung kann insbesondere zur Trennung von zwei Teilchenarten mit verschiedenen Polarisierbarkeiten verwendet werden.

Experimentelle Ergebnisse haben gezeigt, daß sich mit einer Sortieranordnung gemäß Fig. 4a Erythrozyten sauber von sogenannten Jurkart-Zellen trennen ließen, ob beide Zellen die gleiche Größe aufweisen.

Falls das Strömungsprofil im Kanal nicht die in Fig. 4a dargestellte ausgeprägt parabolische Gestalt, sondern eine Plateauform besitzt, so werden Sortierelektroden 41c, 41d gemäß Fig. 4b vorgesehen. Die Strömungsgeschwindigkeit steigt vom Kanalrand her zunächst an und bleibt dann in einem mittleren Bereich des Kanals im wesentlichen konstant. Zur Erzielung einer linearen Sortierwirkung besitzt die Sortierelektrode 41a im mittleren Bereich eine gerade Bandform und an den Enden Krümmungen zur Berücksichtigung der sich ändernden Antriebskraft Fₛ. Für eine nichtlineare Sortierwirkung ist die Sortierelektrode 41d gekrümmt. Vom Ansatz der Sortierelektrode 41d am Steueranschluß 14 hin zu deren Ende ergibt sich eine zunehmende Wirkung der Feldbarriere.

Die Gestalt der Sortierelektroden kann auch an kompliziertere Strömungsprofile angepaßt werden, wie dies in Fig. 4c gezeigt ist. Im Mikrosystem 20 münden ein erster Kanal 211 mit einer hohen Strömungsgeschwindigkeit und ein zweiter Kanal 212 mit einer geringeren Strömungsgeschwindigkeit in einen gemeinsamen Kanal 21. Aufgrund der Laminarität der Strömung bleibt das Strömungsprofil auch im gemeinsamen Strömungsverlauf zunächst erhalten. Entsprechend sind die Sortierelektroden 41e bzw. 41f zur Erzielung einer bestimmten linearen oder nichtlinearen Sortierwirkung gekrümmt ausgebildet. Je geringer die Strömungsgeschwindigkeit, desto größer ist der Anstellwinkel zwischen der Richtung der Mikroelektrode (Ausrichtung der Bezugsfläche) und der Kanallängsrichtung (y-Richtung).

In den Fig. 4b und 4c ist aus Übersichtlichkeitsgründen die Fokussierelektrode 17 gemäß Fig. 4a nicht dargestellt.

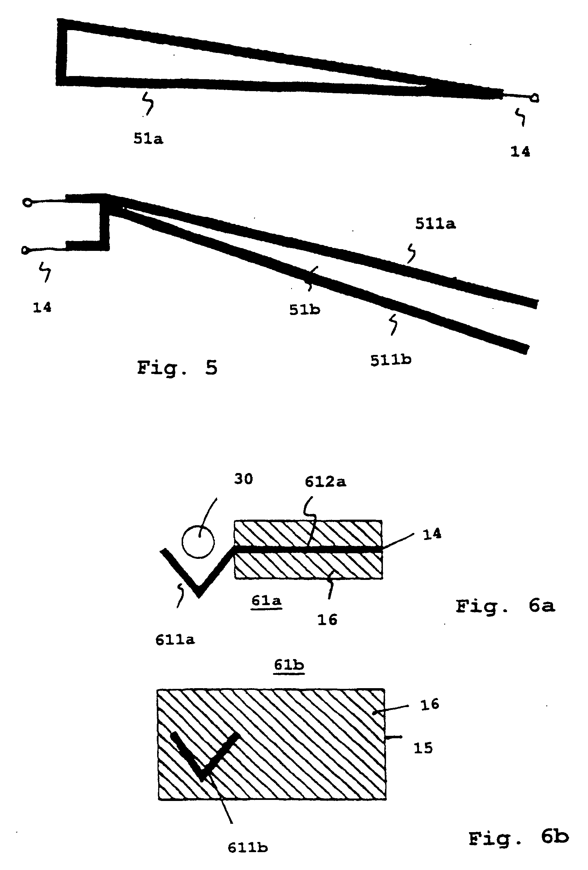

Die oben erläuterte Sortierung erfolgt unter der Annahme eines über die gesamte Mikroelektrodenlänge konstanten Potentials. In der Realität treten jedoch geringfügige elektrische Verluste entlang der Mikroelektrode auf, so daß die Feldbarriere vom Ansatz der Mikroelektrode (bei der Steuerleitung) hin zu ihrem Ende immer kleiner wird. Diese Erscheinung kann bei der Krümmung der Sortierelektroden berücksichtigt werden, indem auf der Steuerleitungsseite des Kanals eine größere Elektrodenkrümmung vorgesehen ist als am Ende der Sortierelektroden. Die genannte Erscheinung kann jedoch auch bewußt für zusätzliche, nicht-lineare Trennwirkungen ausgenutzt werden. Der Potentialabfall hin zum Ende der Mikroelektroden kann bei abgewandelten Ausführungsformen speziell durch Maßnahmen zur Ausbildung von Feldgradienten verstärkt werden. Dies bedeutet, daß die Höhe der durch die Mikroelektrode gebildeten Feldbarriere im Verlauf des gekrümmten Elektrodenbandes zu- oder abnimmt. Derartige Gradientenelektroden können mit einer Gestalt gemäß Fig. 5 aufgebaut sein.

Zur Teilchensortierung in Bezug auf verschiedene Merkmalsgruppen können mehrere Sortierelektroden gemäß Fig. 4 in Kanalrichtung aufeinanderfolgend angeordnet werden. Jede Sortierelektrode ist mit einem charakteristischen Potential oder Potentialverlauf bei einer vorbestimmten Frequenz beaufschlagt. So können beispielsweise relativ niedrige Frequenzen (im Bereich von rd. 10 kHz) zur Sortierung in Bezug auf verschiedene dielektrische Membraneigenschaften und hohe Frequenzen (oberhalb 100 kHz) zur Sortierung in Abhängigkeit von der zytoplasmatischen Leitfähigkeit von biologischen Zellen verwendet werden.

Fig. 5 zeigt Gradientenelektroden 51a, 51b aus Übersichtlichkeitsgründen mit geraden Elektrodenbändern. Zur Einstellung erfindungsgemäß ausgebildeter Feldbarrieren mit gekrümmten Bezugsfiächen besitzen die dargestellten Gradientenelektroden zusätzlich noch eine charakteristische, anwendungsabhängige Krümmung entsprechend den oben erläuterten Prinzipien.

Die Gradientenelektrode 51a wird durch ein geschlossenes, um eine dreieckige Fläche geführtes Elektrodenband gebildet. Mit zunehmendem Abstand von der Steuerleitung 14 wird die Feldliniendichte entsprechend der Auffächerung des Dreiecks gering. Entsprechendes gilt für die Gradientenelektrode 51b mit zwei divergierenden Teilbändern 511b und 512b.

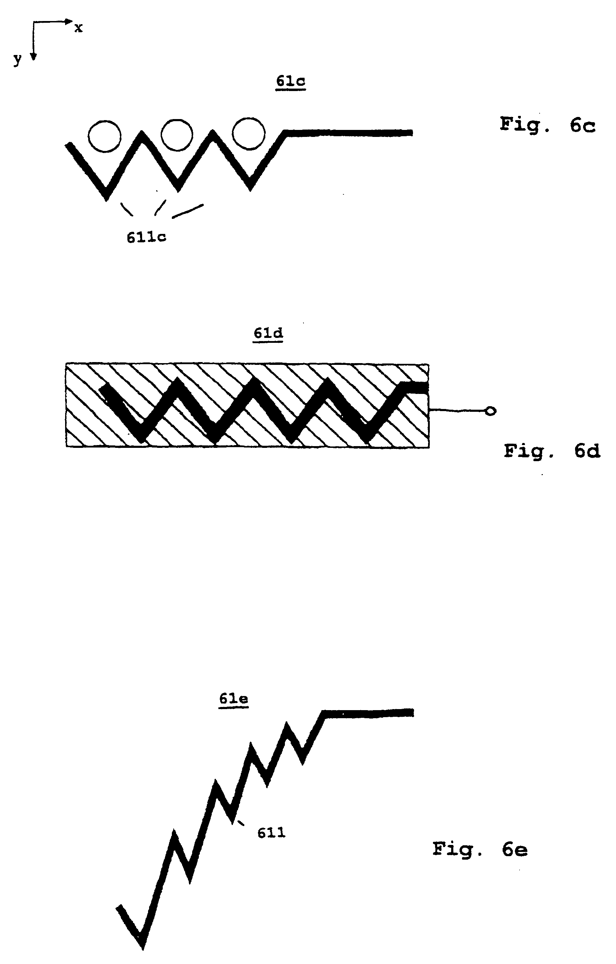

Eine weitere wichtige Anwendung fluidischer Mikrosysteme besteht im Sammeln und zumindest zeitweisen Anordnen von Teilchen oder Teilchengruppen im suspensionsflüssigkeitsdurchströmten Kanal. Hierzu werden erfindungsgemäße Elektrodenanordnungen als Fangelektroden gestaltet, wie dies im folgenden unter Bezug auf die Fign. 6 bis 8 erläutert wird.

Fig. 6a zeigt die Grundform einer Fangelektrode. Wiederum ist lediglich eine Mikroelektrode auf der Boden- oder Deckfläche eines Kanals gezeigt, die mit einer zweiten Mikroelektrode auf der gegenüberliegenden Kanalseite zusammenwirkt. Eine Fangelektrode 61a besteht aus einem Elektrodenband mit einem Winkelabschnitt 611a und einem Zuführungsabschnitt 612a. Der Winkelabschnitt 611a bildet einen in Strömungsrichtung (x-Richtung) weisenden Winkel. Der Öffnungswinkel des Winkelabschnittes 611a wird in Abhängigkeit von der Gestalt der einzufangenden Teilchen gewählt und ist vorzugsweise kleiner als 90°, z.B. im Bereich von 20 bis 60°. Die gegenüberliegenden Winkelabschnitte zusammenwirkender Elektroden bilden eine für die einzufangenden Teilchen 30 auch unter Wirkung der Antriebskraft durch die Strömung nicht passierbare Barriere. Diese Barriere bleibt für die Dauer der Ansteuerung der Fangelektroden erhalten. Der Zuführabschnitt 612a ist durch eine Isolationsschicht 16 elektrisch unwirksam. Fig. 6b zeigt eine abgewandelte Form einer Fangelektrode 61b, die entsprechend der oben unter Bezug auf Fig. 3 erläuterten Abdecktechnik hergestellt ist. Der elektrisch wirksame Winkelabschnitt 611b wird durch eine Ausnehmung in der Isolationsschicht 16 gebildet, durch die eine tieferliegende metallische Schicht 15 hin zur Suspensionsflüssigkeit mit den Teilchen offen liegt.

Die Fign. 6c und 6d zeigen entsprechende Fangelektroden 61c und 61d jeweils mit einer Vielzahl von Winkelabschnitten 611c bzw. 611d. Diese Winkelabschnitte sind wiederum zum Auffangen anströmender Partikel 30 eingerichtet. Durch die Aneinanderreihung der Winkelabschnitte 611c bzw. 611d quer zur Kanallängsrichtung (x-Richtung) können die in den verschiedenen Kanalbereichen anströmenden Teilchen selektiv aufgefangen werden. Eine Fangelektrode 61c bzw. 61d wird vorteilhafterweise mit einer der Sortierelektroden gemäß den Fign. 4a bis 4c kombiniert. Die sortierten Teilchen werden separat in den einzelnen Fangbereichen der Fangelektroden aufgefangen. Die Fangelektrode 61d entspricht im wesentlichen der Fangelektrode 61c, wobei die genannte Abdecktechnik implementiert wurde.

Die Fangelektroden 61c bzw. 61d sind besonders gut geeignet eine Aufreihung von Teilchen in der Suspensionsströmung nach Art einer Startlinie zu bilden, von der die Teilchen bei Abschalten der Steuerpotentiale der Fangelektroden simultan fortströmen.

Fig. 6e zeigt eine weitere Ausführungsform einer Fangelektrode 61e, bei der auch eine Vielzahl von Winkelabschnitten 611e vorgesehen sind, die jedoch für die Sammlung bzw. das Auffangen verschieden großer Teilchen oder verschieden großer Ansammlungen aus diesen eingerichtet sind.

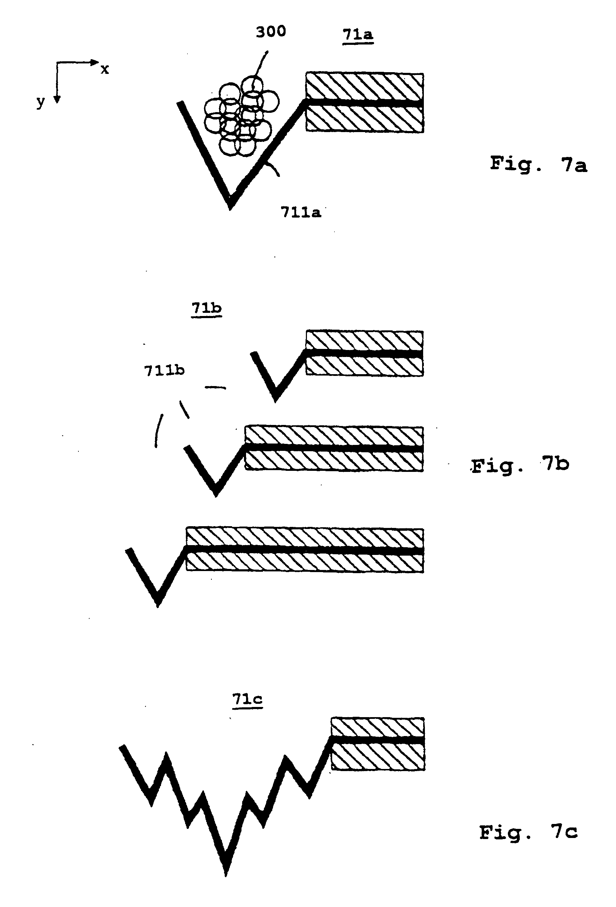

Die Ansammlung einer Teilchengruppe 300 mit einer Fangelektrode 71a ist in Fig. 7a illustriert. Diese Ausführungsform einer Fangelektrode unterscheidet sich von der Fangelektrode gemäß Fig. 6a lediglich durch die Ausmaße. Diese Gestaltung eignet sich besonders gut zur Bildung von Teilchenaggregaten. Wiederum wird eine Kombination mit einer Sortieranordnung gemäß den Fign. 4a bis 4c bevorzugt realisiert.

Die Elektrodenanordnung gemäß Fig. 7b ist zum separaten Auffangen von Teilchen oder Teilchengruppen aus der Suspensionsströmung im Kanal eingerichtet, die sich in Bezug auf ihre Strömungsbahn in x-Richtung unterscheiden. Die Mikroelektrodenanodnung 71b umfaßt mehrere Teil-Fangelektroden jeweils mit einem Winkelabschnitt 711b, die separat ansteuerbar sind. Bei Kombination einer derartigen Fangelektrodenanordnung mit einer Sortieranordnung gemäß den Fig. 4a bis 4c kann mit besonderem Vorteil die folgende Verfahrensweise realisiert werden.

Zunächst wird ein Teilchengemisch, das durch den Kanal im Mikrosystem strömt, in Abhängigkeit von den passiven elektrischen Eigenschaften der Teilchen sortiert und somit auf verschiedene, in x-Richtung voneinander beabstandete Bahnen gelenkt. Dann erfolgt die teilchenartspezifische Sammlung der in den einzelnen Bahnen anströmenden Teilchen mit einer Fangelektrode gemäß Fig. 7b. Durch eine zeitlich aufeinanderfolgende Freigabe der Teil-Fangelektroden (jeweils durch Abschalten der Steuerpotentiale) können die vorher sortierten Teilchen gruppenweise im Mikrosystem weitergeströmt werden. Im weiteren Kanalverlauf kann beispielsweise eine Aufspaltung in mehrere Teilkanäle erfolgen, in die die Gruppen der Teilchenarten spezifisch gelenkt werden.

Fig. 7c zeigt eine weitere Fangelektrode 71c zur Erzeugung einer vorbestimmten Partikelformation.

Die Winkelabschnitte der in den Fign. 6 und 7 gezeigten Fangelektroden können sich anwendungsabhängig über die gesamte Kanalbreite oder nur über Teile des Kanals erstrecken. Innerhalb einer Elektrodenanordnung können Fangelektroden für einzelne Teilchen und/oder für Teilchengruppen vorgesehen sein.

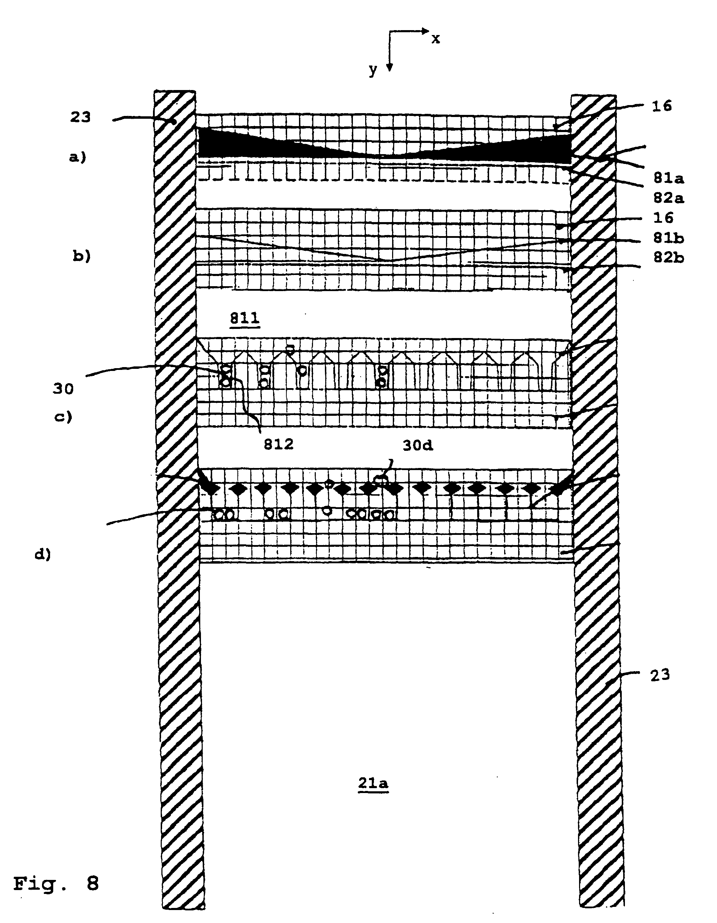

Weitere Ausführungsformen kombinierter Sortier- und Fangelektroden sind in der Draufsicht auf die Bodenfläche 21a eines durch die Spacer 23 begrenzten Kanals gemäß Fig. 8 illustriert. Der Kanal wird in y-Richtung von der Suspensionsflüssigkeit mit suspendierten Teilchen durchströmt. Gemäß Fig. 8a wirkt eine flächige Mikroelektrode 81a auf der Bodenfläche 21a mit einer geraden, bandförmigen Mikroelektrode 82a (gestrichelt eingezeichnet) auf der entgegengesetzten Deckfläche des Kanals zusammen. Die flächige Mikroelektrode 81a ist durch die oben erläuterte Abdecktechnik hergestellt. Eine metallische Schicht trägt eine Isolationsschicht 86 mit einer Ausnehmung entsprechend der Gestalt der Mikroelektrode 81a (schwarz gezeichnet). Die Feldlinien zwischen den Mikroelektroden 81a und 82a verlaufen quer zur Strömungsrichtung in inhomogener Weise, so daß sich eine asymmetrische Feldbarriere bzw. wiederum eine erfindungsgemäß gekrümmte Bezugsfläche ergibt. In Kanalmitte ist die Feldliniendichte am größten, so daß auch die elektrisch erzeugten Kräfte im Bereich der höchsten Strömungsgeschwindigkeit liegen. Dadurch wird in x-Richtung quer über die Kanalbreite ein im wesentlichen konstantes Gleichgewicht zwischen der Antriebskraft durch die Strömung und der elektrischen Polarisationskraft ausgebildet. Gemäß Fig. 8b wird wiederum eine Feldbarriere mit gekrümmter Bezugsfläche gebildet. Die Mikroelektroden 81b, 82b sind beide linear oder bandförmige ausgeführt und nicht gegenüberliegend, sondern versetzt zueinander angeordnet.

Eine Elektrodenanordnung zur Bildung von Teilchenaggregaten ist in Fig. 8c gezeigt. Die Mikroelektroden 81c, 82c bilden eine Reihe nebeneinander angeordneter, trichterförmiger Partikelfänger. Jeder Partikelfänger 11 wird durch eine Feldbarriere gebildet, die sich in Strömungsrichtung zunächst trichterförmig verengt und dann in einen geraden Kanalabschnitt 812 mündet. Der Kanalabschnitt ist so bemessen, daß zwei Teilchen in Strömungsrichtung hintereinander angeordnet werden können. Durch Ausbildung von Adhäsionskräften bilden die Partikel ein Aggregat (sogenannte Pärchenbeladung in Strömungsrichtung). Die Ausführungsform gemäß Fig. 8d ist dahingehend abgewandelt, daß eine Pärchenbeladung quer zur Strömungsrichtung erfolgt. Dabei sind die einzelnen Fängerelemente 811d mit eingangsseitigen Elektrodenspitzen 813d ausgebildet, mit denen eine zusätzliche Barrierewirkung oder Filterwirkung erzielt wird und bereits vorhandene Aggregate oder größere Teilchen 30d von einer Anordnung in der Fangelektrode 81d ausgeschlossen werden.

Eine weitere Ausführungsform einer erfindungsgemäßen Elektrodenanordnung gemäß der obengenannten zweiten Variante ist in Fig. 9 dargestellt. Im Mikrosystem 20 ist zwischen den Spacern 23 ein Kanal 21 gebildet, der durch eine Trennwand 231 in Teilkanäle 211 und 212 unterteilt ist. Die Trennwand 231 besitzt eine Öffnung 232, in deren Bereich an den Seitenflächen des Kanals 21 die Mikroelektroden 91 und 92 angebracht sind.

Die Mikroelektroden 91 und 92 sind sogenannte dreidimensionale oder hohe Elektroden, die an den Seitenflächen aus den Ebenen der Boden- und Deckflächen des Kanals 21 herausragen. Die Herstellung der Mikroelektroden 91 und 92 erfolgt mit an sich bekannten Techniken der Halbleiterprozessierung (z.B. mit dem LIGA-Verfahren). Die Mikroelektrode 91 ist flächig ausgeführt. Die Feldlinien reichen zur gegenüberliegenden, bandförmig ausgeführten Mikroelektrode 92 und bilden damit einen gekrümmten Fangbereich mit der in Fig. 1c illustrierten Bezugsfläche.

Werden die Mikroelektroden 91, 92 mit elektrischen Hochfrequenzpotentialen angesteuert, so werden die Partikel 30 mit negativer Dielektrophorese durch die Öffnung 232 in den benachbarten Teilkanal gedrückt. Diese Teilchenablenkung kann wiederum selektiv in Abhängigkeit von den passiven elektrischen Eigenschaften der suspendierten Teilchen erfolgen. Teilchen mit geringer Polarisierbarkeit bleiben im Ausgangskanal, während Teilchen mit hoher Polarisierbarkeit in den Nachbarkanal abgelenkt werden.

Bei der Gestaltung gemäß Fig. 9 ist es nicht zwingend erforderlich, daß die Mikroelektrode 91 beschaltet ist. Sie kann erdfrei geschaltet sein (floating) oder auch ganz weggelassen werden. Im letzteren Fall wirkt die Mikroelektrode 92 als Antenne. Die Mikroelektroden 91, 92 erstrecken sich vorzugsweise über die gesamte Höhe der Seitenflächen des Kanals.

Ein Ausführungsbeispiel einer Elektrodenanordnung entsprechend der obengenannten dritten Variante ist in Fig. 10 (entsprechend Fig. 1d) illustriert. In einem Mikrosystem verlaufen wiederum zwei Teilkanäle 211, 212 parallel zueinander und durch eine Trennwand 231 mit einer Öffnung 232 voneinander getrennt. Die Elektrodenanordnung besteht aus den Mikroelektroden auf den Boden- und Deckflächen in Form von Fokussierungselektroden 101, 102 und der Hilfselektrode 103. Die Hilfselektrode ist an der Trennwand 231 an die Öffnung 232 angrenzend an der stromabwärts gelegenen Seite der Öffnung 232 angeordnet. Die Hilfselektrode 103 verfügt nicht über eine Steuerleitung. Sie dient lediglich der Formung der Bezugsfläche der durch die Elektrodenanordnung gebildeten Feldbarrieren. Die Mikroelektroden wirken wie folgt zusammen.

Die Fokussierelektroden 101 und 102 dienen jeweils der Fokussierung der in den Teilkanälen 211 bzw. 212 anströmenden Teilchen 30a, 30b auf eine Mittellinie entsprechend der Position der Öffnung 232 in der Trennwand 231. Analog zum unter Bezug auf Fig. 9 erläuterten Ablenkprinzip werden die Teilchen durch die Feldbarriere zwischen der Fokussierelektrode 101 und der Hilfselektrode 103 bzw. zwischen der Fokussierelektrode 102 und der Hilfselektrode 103 durch die Öffnung 232 in den benachbarten Teilkanal abgelenkt oder im gegebenen Teilkanal belassen. Gemäß einer bevorzugten Verfahrensweise werden die Fokussierelektroden 101, 102 mit verschiedenen Frequenzen betrieben, um teilchenselektiv zu wirken. Demtentsprechend ist wiederum eine selektive Teilchensortierung in die Teilkanäle oder eine Ablenkung vorbestimmter Teilchen in einen benachbarten Teilkanal zur Durchführung einer bestimmten Wirkstoffbehandlung mit der dort gegebenen Suspensionsflüssigkeit erzielbar.

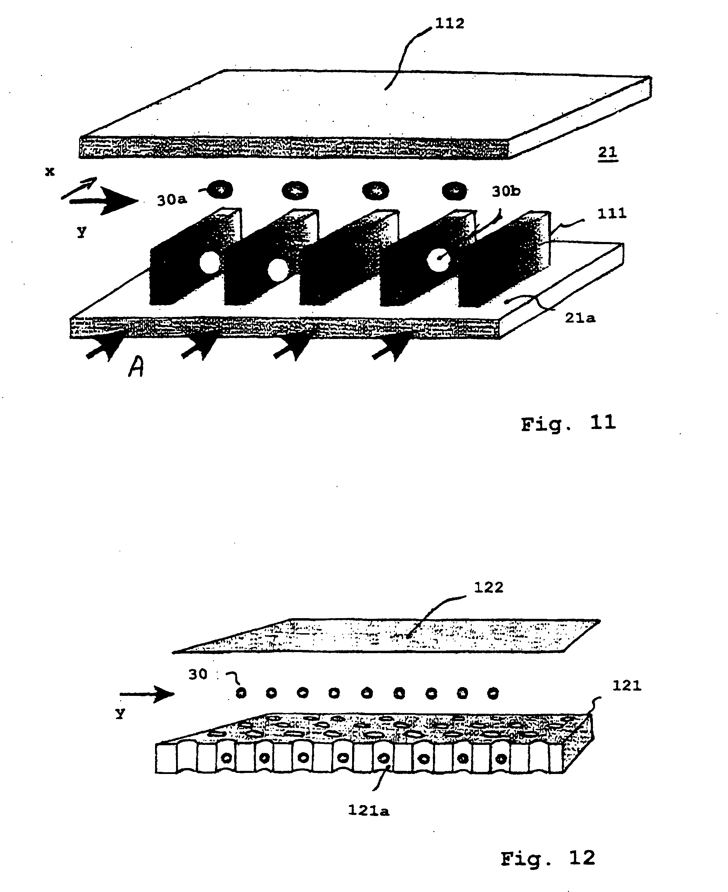

Eine weitere dreidimensionale Elektrodenanordnung ist in Fig. 11 dargestellt. Ein Kanal 21 wird in y-Richtung mit einer Suspensionsflüssigkeit durchströmt. Auf der Bodenfläche 21a ist eine Gruppe von Mikroelektroden 111 angeordnet, die in den Kanal 21 hineinragen und voneinander beabstandet in Strömungsrichtung (y-Richtung) ausgerichtet sind. Jede Mikroelektrode 111 besitzt die Form eines Quaders. Die Mikroelektroden 111 bestehen aus Metall oder besitzen eine metallische Oberflächenbeschichtung, ohne selbst mit einer Steuerleitung versehen zu sein.

Auf der gegenüberliegenden Kanalwand (Deckfläche, nicht dargestellt) ist eine flächige Elektrodenanordnung 112 (Ablenkelektrode) vorgesehen, die mit den Mikroelektroden 111 wie folgt zusammenwirkt. Die in y-Richtung strömenden Teilchen 30a werden den Feldbarrieren ausgesetzt, die durch die feldformenden Mikroelektroden 111 asymmetrisch und durch gekrümmte Bezugsflächen gekennzeichnet sind. Wiederum erfolgt eine Ablenkung der Teilchen in Abhängigkeit von den passiven elektrischen Eigenschaften. Schwach polarisierbare Teilchen 31a strömen weiter in y-Richtung, während stärker polarisierbare Teilchen 30b in die Abstände zwischen den feldformenden Elektroden 111 abgelenkt werden. Die abgelenkten Teilchen 30b werden dementsprechend aufgefangen oder aufgesammelt und nicht mehr mit der Strömung in y-Richtung weiter transportiert.

Gemäß einer bevorzugten Ausführungsform ist die Elektrodenanordnung gemäß Fig. 11 an einer Kreuzung von zwei Kanälen vorgesehen. Der Kanal 21 in y-Richtung wird von einem (nicht dargestellten) Kanal gekreuzt, durch den eine Suspensionsflüssigkeit in x-Richtung (Pfeile A) strömt. Diese laterale Zusatzströmung transportiert die abgelenkten Teilchen 30b kontinuierlich aus den Zwischenräumen zwischen den feldformenden Elektroden 111 in den Querkanal.

Die Geometrie der feldformenden Mikroelektroden 111 kann an die Strömungsverhältnisse und den Feldverlauf in den Elektrodenzwischenräumen und die Gestalt der gegenüberliegenden Elektrodenanordnung 112 angepaßt sein.

Die Ausführungsform gemäß Fig. 11 kann durch Bereitstellung einer volumenförmigen feldformenden Elektrode 121 anstelle der feldformenden Elektroden 111 gemäß Fig. 12 modifiziert werden. Die volumenförmige Mikroelektrode wird auch als Sammelelektrode 121 bezeichnet. Die Sammelelektrode 121 befindet sich beispielsweise auf der Bodenfläche eines Kanals (nicht dargestellt) und besteht aus einem quaderförmigen Block aus metallischem oder metallisch beschichtetem Material mit einer Vielzahl von spalten- und reihenweise angeordneten Bohrungen oder Reservoiren 121a. Die Sammelelektrode ist auf der Vorderseite geschnitten dargestellt, so daß die Reservoire 121a erkennbar sind. Die Sammelelektrode 121a wirkt wie folgt mit der flächigen Elektrodenanordnung 122 (Ablenkelektrode) auf der gegenüberliegenden Kanalwand zusammen. Zwischen den Mikroelektroden 122, 121 wird eine asymmetrische Feldbarriere erzeugt, die dazu eingerichtet ist, selektiv Teilchen in die Reservoire 121a abzulenken. Die Teilchen 30 strömen y-Richtung durch den Kanal. Teilchen, die durch die Feldwirkung nach unten in die Sammelelektrode 121 abgelenkt werden, gelangen in die Reservoire 121a und werden dort fixiert. Nachdem sämtliche Reservoire 121a gefüllt sind, kann die Ansteuerung der Elektrodenanordnung derart erfolgen, daß die Teilchen simultan aus den Reservoiren 121a in die Strömung überführt und in dieser als Teilchen- oder Aggregatformation weiter transportiert werden. Hierzu kann gegebenenfalls unterhalb der Sammelelektrode 121 eine weitere flächige Elektrodenanordnung (nicht dargestellt) vorgesehen sein, die im wesentlichen wie die flächige Elektrodenanordnung 122 ausgebildet ist.

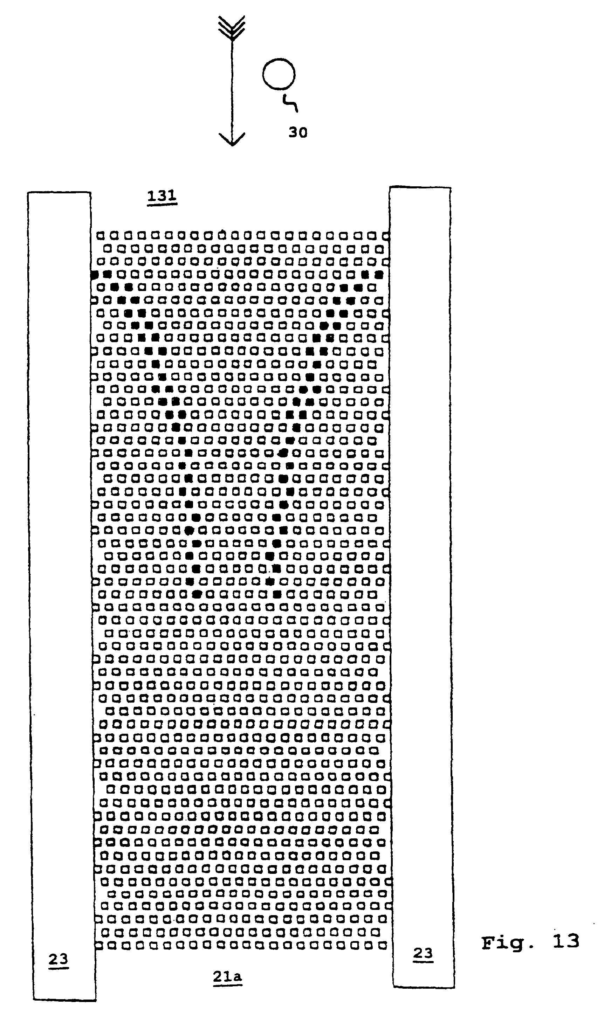

Gemäß einem besonderen Gesichtspunkt der Erfindung können die Mikroelektroden bei den einzelnen Gestaltungsformen an sich segmentiert sein. In diesem Fall besteht jede Mikroelektrode aus einer Reihe von Elektrodensegmenten, die entsprechend der gewünschten Elektrodenfunktion angeordnet sind. Eine besonders vielseitig einsetzbare Mikroelektrode 131 ist in Fig. 13 als Array einer Vielzahl von matrixartig angeordneten, pixelförmigen Elektrodensegmenten dargestellt. Die Elektrodensegmente sind über der gesamten Breite der Bodenfläche 21a zwischen den Spacern 23 angeordnet und einzeln ansteuerbar. Dies ermöglicht die Ausbildung der gewünschten gekrümmten Feldbarrieren, insbesondere entsprechend der obengenannten ersten Variante, in Abhängigkeit von der konkreten Anwendung, insbesondere in Abhängigkeit von den jeweils zu manipulierenden Teilchen, den Strömungsverhältnissen und der Aufgabe des Mikrosystems. In Fig. 13 sind die momentan angesteuerten Pixel schwarz und die nicht-angesteuerten Pixel weiß gezeichnet. In diesem Fall übernimmt die segmentierte Mikroelektrode 131 die Funktion eines Partikeltrichters gemäß Fig. 2, mit dem die Teilchen 30 in die Kanalmitte fokussiert werden.

Die pixelförmigen Elektrodensegmente ermöglichen eine verlustminimierende Fokussierung, Sortierung oder Sammlung von Teilchen. Jedes Elektrodensegment kann mit einem eigenen Potentialwert (Spannung) bzw. einer eigenen Frequenz ansgesteuert werden. Damit läßt sich ein beliebig vorzugebendes dielektrisches Kraftfeld entlang des Kanales ausbilden. So z.B. läßt sich der Einfluß des Strömungsprofiles dadurch kompensieren, daß die quer zur Kanallängsrichtung angeordneten Pixel mit einer Spannung entsprechend der Quadratwurzel des Profils der Strömungsgeschwindigkeit angesteuert werden.

Die Größe der Elektrodensegmente und Abstände zwischen den Elektrodensegmenten sind vorzugsweise kleiner als charakteristische Dimensionen der zu manipulierenden Teilchen, können aber auch größer sein.

Sämtliche Teilchenmanipulierungen erfolgen berührungsfrei, so daß sich die Mikrosysteme besonders für die Manipulierung biologischer Zellen oder Zellbestandteile eignen.

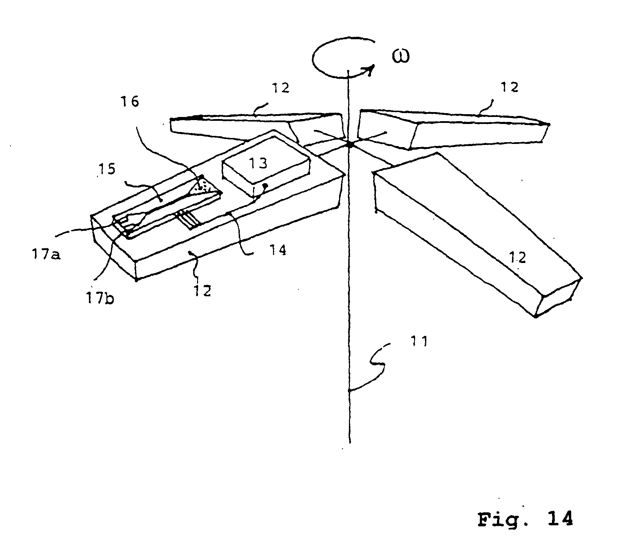

Die Figuren 14 bis 16 zeigen eine schematische Perspektivansicht eines Aufbaus einer Zentrifuge mit einem Mikrosystem, eine schematische Draufsicht auf ein Mikrosystem, das zur Teilchentrennung eingerichtet ist, und eine schematische Draufsicht auf ein programmierbares Beladungsmikrosystem.

Die hier beschriebenen Ausführungsformen der Erfindung beziehen sich auf die Kombination eines Mikrosystems, das mit einer Mikroelektrodeneinrichtung zur Ausübung negativer oder positiver Dielektrophorese ausgestattet ist (dielektrophoretisches Mikrosystem), mit einer Schwingrotorzentrifugeneinrichtung. Sowohl das dielektrophoretische Mikrosystem (abgesehen von der mindestens einseitigen Verschließbarkeit von Kanalstrukturen) als auch die Schwingrotorzentrifugeneinrichtung sind jeweils an sich bekannt, so daß auf deren technische Einzelheiten hier nicht weiter eingegangen wird. Es wird betont, daß der Begriff der Schwingrotorzentrifugeneinrichtung hier auch im weitesten Sinne dahingehend zu verstehen ist, daß jede Zentrifugeneinrichtung mit mindestens einem drehzahlabhängig aufrichtbaren Rotor eingeschlossen ist, der selbst das Mikrosystem und die zugehörige Steuerung bildet, in den das Mikrosystem und die zugehörige Steuerung integriert oder auf den das Mikrosystem und die zugehörige Steuerung aufgesetzt sind.

Die manipulierten Partikel können synthetische Teilchen oder biologische Objekte umfassen. Die synthetischen Teilchen sind beispielsweise membranumhüllte Gebilde, wie Liposomen oder Vesikeln, oder sogenannte Beads oder auch Makromoleküle. Die biologischen Objekte umfassen beispielsweise biologische Zellen oder Bestandteile von diesen (z.B. Zellorganellen), Bakterien oder Viren. Die Partikel können auch Aggregate oder Zusammenballungen derartiger Teilchen und/oder Objekte sein. Vorzugsweise werden zellphysiologisch oder medizinisch relevante Fluide mit Leitfähigkeiten unterhalb 5 Siemens/m verwendet.

Fig. 14 ist eine schematische Übersichtsdarstellung einer Vorrichtung zur Illustration der Anbringung eines dielektrophoretischen Systems an einer Zentrifugeneinrichtung.

An einem üblichen oder anwendungsabhängig modifizierten Rotor einer Zentrifuge mit der Drehachse 11 befinden sich vier Aufnahmen 12, in die jeweils paßgerecht und für die applizierten Drehzahlen entsprechend ein Mikrosystem 15 und eine Steuerelektronik 13 zur Ansteuerung des Mikrosystems mit hochfrequenten Wechselsignalen verschiedener Phasenlage und Amplitude eingesetzt sind. Die Steuerelektronik ist über Kabel 14, Stecker oder anderweitig mit dem Mikrosystem 15 verbunden. Die Energieversorgung der Steuereinrichtung erfolgt vorzugsweise über eine elektrische Verbindung, (umlaufender Kontakt) mit dem festen Laborsystem. Das Mikrosystem hat ein Eingangsdepot 16, das anwendungsabhängig verschieden groß ausgelegt sein kann und vor der Zentrifugation mit einer Teilchen- oder Zellsuspension gefüllt wird. Vom Eingangsdepot 16 aus verläuft eine Kanalstruktur, deren Einzelheiten weiter unten erläutert werden, bis zu Auffangzonen 17a, 17b, die ein zumindest während des Zentrifugierens geschlossenes Ende des Mikrosystems 15 bilden. Dies bedeutet, daß das Ende des Mikrosystems entweder dauerhaft abgeschlossen oder bei Stillstand der Vorrichtung durch entsprechende Verbindungselemente geöffnet und an vorbestimmte Zusatzsysteme zur Probenübertragung angeschlossen werden kann. Das Mikrosystem 15 ist so auf der Aufnahme 12 angeordnet, daß bei Betrieb der Zentrifugeneinrichtung (Drehung des Rotors um die Drehachse 11 mit der Drehfrequenz ω) die auf das Mikrosystem 15 und in diesem befindliche Partikel wirkenden Zentrifugalkräfte in der Bezugsrichtung vom Eingangsdepot 18 hin zu den Auffangzonen 17a, 17b gerichtet sind. Die Aufnahmen 12 sind verschwenkbar am Rotor (nicht dargestellt) angebracht. Beim Stillstand der Zentrifuge sind die Aufnahme 12 im wesentlichen vertikal oder mit einem geringen Winkel gegenüber der Drehachse ausgerichtet. Beim Zentrifugenbetrieb richten sich die Aufnahmen 12 drehzahlabhängig in einen größeren Winkel bis hin in die horizontale Ausrichtung senkrecht zur Drehachse 11 auf. Unter der Wirkung der Gravitationskraft (bei Stillstand der Zentrifuge) bwz. der Zentrifugalkräfte durchlaufen die Teilchen das elektronisch gesteuerte Mikrokanalsystem und sammeln sich in den Auffangzonen (z.B. am geschlossenen Ende des von der Rotorachse wegweisenden Teils des Mikrosystems).

Bei diesem Durchlauf werden die Partikel nach vorbestimmten Programmen (s. unten) behandelt. Da die Teilchen in Abhängigkeit von ihrer Dichte verschiedene Bewegungen ausführen und Endpositionen einnehmen, wird der Vorteil der Zentrifugaltrennung und -bewegung mit den Möglichkeiten der programmierbaren Dielektrophorese kombiniert. In der Regel wird negative Dielektrophorese, in Ausnahmefällen auch positive Dielektrophorese der Teilchen genutzt. Ein weiterer Vorteil ist die Steuerung der Teilchenbewegung über die Rotationsgeschwindigkeit (ω) des Rotors 11. Da hierbei ebenfalls programmierbare Variationen durchlaufen werden können, ist ein zweiter Komplex von festlegbaren Parametern bei der Partikelmanipulation gegeben.

Die Zentrifugeneinrichtung ist mit einer (nicht dargestellten) Drehzahlsteuerung versehen, die für eine reproduzierbare und genaue Drehzahleinstellung insbesondere in niedrigen Drehzahlbereichen eingerichtet ist. Die Drehzahl wird anwendungsabhängig je nach der gewünschten Geschwindigkeit der zu manipulierenden Teilchen und in Abhängigkeit vom konkreten Zentrifugenaufbau gewählt. Die interessierenden Partikelgeschwindigkeiten liegen für biologische Partikel (z.B. Zellen) unterhalb von rd. 500 µm/s (vorzugsweise im Bereich von 50 bis 100 µm/s) und für synthetische Partikel (z.B. Latex-Beads) bei höheren Geschwindigkeiten (z.B. einige mm/s). Die Drehzahl der Zentrifugeneinrichtung wird entsprechend den Zusammenhängen von Drehzahl und Zentrifugalkraft in Abhängigkeit von der Größe bzw. Massendichte der Partikel gewählt. Die folgenden Angaben beziehen sich auf einen Abstand des Mikrosystems von der Rotorachse im Bereich von 1 bis 10 cm. Für Partikeldurchmesser im Bereich von 50 bis 600 nm (z.B. Viren) können die Drehzahlen beispielsweise im Bereich von 1 bis 1000 U/min liegen. Bei Partikeln mit einem Durchmesser von rd. 5 µm werden Drehzahlen bis zu 100 U/min bevorzugt, wobei jedoch auch höhere Drehzahlen einstellbar sind. Bei besonders kleinen Partikeln, z.B. Makromoleküle sind auch noch höhere Drehzahlen realisierbar. Für biologische Zellen ergeben sich bei einem Abstand des Mikrosystems von rd. 5 bis 10 cm von der Drehachse 11 Drehzahlen im Bereich von wenigen Umdrehungen pro Minute bis zu einigen 100 (z. B. 600) Umdrehungen pro Minute, vorzugsweise unterhalb 100 U/min. Die erzielbaren Zentrifugalkräfte liegen im Bereich von pN bis nN. Die Zentrifugeneinrichtung ist jedoch auch für größere Drehzahlen ausgelegt, die insbesondere für kleine Partikel oder für Reinigungsoder Spülzwecke eingestellt werden können. Diese erhöhten Drehzahlen können bis zum Bereich der Drehzahlen herkömmlicher Laborzentrifugen reichen.

Die Drehzahl der Zentrifuge wird auch in Abhängigkeit von den dielektrophoretischen Kräften ausgewählt, die auf die Partikel im Mikrosystem wirken. Die dielektrophoretischen Kräfte sind als Polarisationskräfte von der Teilchenart und -größe abhängig. Die Drehzahl wird vorzugsweise so ausgewählt, daß die Zentrifugalkräfte auf die Partikel kleiner oder gleich den dielektrophoretischen Kräften sind. Falls diese nicht bekannt sind, kann die Drehzahl auch in Bezug auf das folgende Kriterium ausgewählt werden. Die Teilchen müssen sich so langsam durch die Kanalstruktur bewegen, daß beim Vorbeitritt an den Mikroelektrodeneinrichtungen genügend Zeit zur dielektrophoretischen Ablenkung bleibt. Die Wirksamkeit oder Unwirksamkeit der dielektrophoretischen Ablenkung in Abhängigkeit von der Drehzahl kann mit geeigneten Sensoren optisch oder elektrisch erfaßt werden.

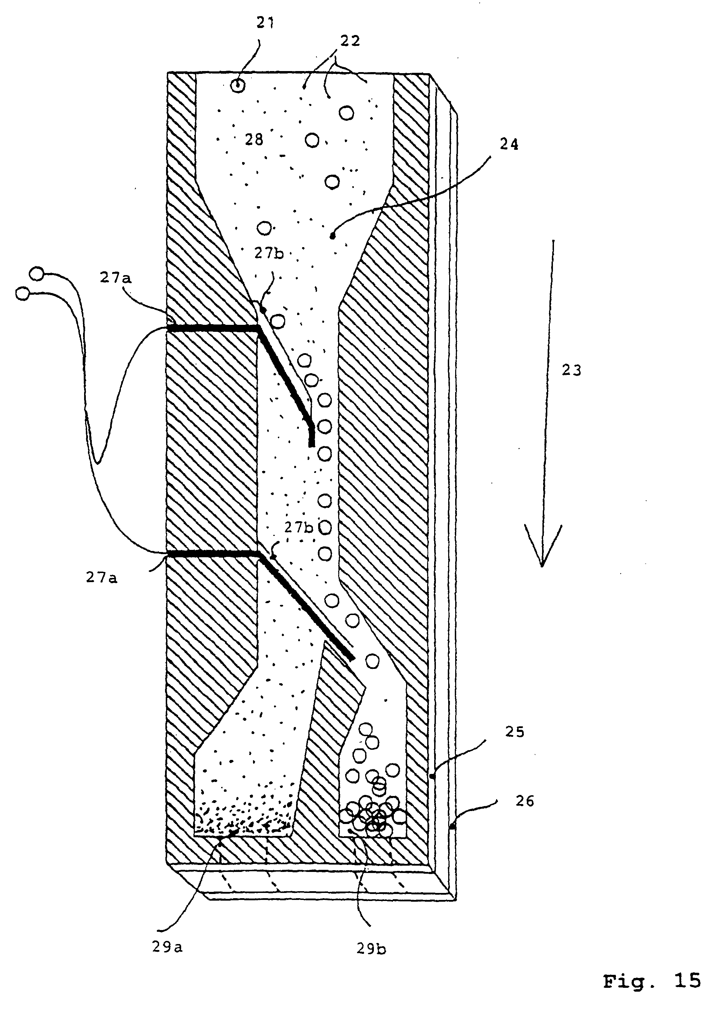

Fig. 15 zeigt in schematischer Weise ein Mikrosystem zur Auftrennung eines Partikelgemisches, bestehend aus größeren Teilchen 21 (z.B. Zellen) und kleinen Teilchen 22, die in einer Suspension vorliegen. Die Zentrifugalkräfte wirken in Pfeilrichtung 23 (Bezugsrichtung). Die typischen Abmessungen der Kanalstruktur 24 sind die folgenden:

| | |
|---|---|
| Breite | einige 10 um bis zu einigen mm (typischerweise: 200 - 400 µm) |
| Länge | einige mm bis zu einigen cm (typischerweise: 20 - 50 mm) |
| Höhe | einige µm bis zu einigen 100 µm (typischerweise: 50 µm) |

Auf der Oberseite 25 und Unterseite 26 des Kanals 24 sind Mikroelektroden 27a, 27b gegenüberliegend angeordnet, die bei Ansteuerung mit einer Wechselspannung (in der Regel einer Frequenz im MHz-Bereich und einer Amplitude von einigen Volt) quer zum Kanal Feldbarrieren erzeugen, die über negative (bedingt auch positive) Dielektrophorese die Teilchen ablenken (im hier gezeigten Fall die großen Teilchen).

Die Kanalstruktur 24 reicht vom Eingangsdepot 28 zu den geschlossenen Kanalenden 29a, 29b, in die sich der in einem mittleren Abschnitt gerade Kanal verzweigt. Ein erstes Paar der Mikroelektroden 27a, 27b ist unmittelbar am kanalseitigen Ende des Eingangsdepots 28 zur Ausbildung einer Feldbarriere angeordnet, die schräg in den Kanal hineinragt und die Aufgabe besitzt, die großen Teilchen 21 in den in Draufsicht rechten Teil des Kanals 24 zu drängen. Ein zweites Paar der Mikroelektroden 27a, 27b ist unmittelbar vor der Verzweigung zu den Kanalenden 29a, 29b angeordnet und bildet eine Feldbarriere, die schräg über die Kanalbreite bis.in die zum Kanalende 29b führende Abzweigung reicht und dazu vorgesehen ist, die großen Teilchen 21 zu diesem Kanalende hin zu führen.

Ein Manipulationsverfahren, das bei diesem Beispiel auf eine Trennung der Teilchen gerichtet ist, umfaßt die folgenden Schritte.

Vor der Zentrifugation wird das Mikrosystem mit einer geeigneten Flüssigkeit gefüllt. Dabei ist das Mikrosystem bereits in eine Aufnahme 12 der Zentrifuge (s. Fig. 14) eingebaut. Der Einbau kann aber auch nach der Befüllung des Mikrosystems erfolgen. Kurz vor Beginn der Zentrifugation werden die Elektroden 27a, 27b angesteuert und im Eingangsdepot 28 wird z.B. mit einer Pipettiereinrichtung die Suspension der zu trennenden Teilchen zugegeben. Die Zentrifugeneinrichtung ist zunächst noch im Ruhezustand, d.h. das Mikrosystem ist vertikal oder zur Vertikalen leicht geneigt ausgerichtet. Die Gravitationskraft, die auf die Teilchen wirkt, führt zu einem masseabhängig verschieden schnellen Absinken in die Kanalstruktur (Sedimentation). Die weitere Bewegung der Teilchen hin zu den Kanalenden erfolgt je nach der gewünschten Teilchengeschwindigkeit ausschließlich unter der Wirkung der Gravitationskraft oder unter der gemeinsamen Wirkung der Gravitationskraft und der Zentrifugalkräfte. Die Zentrifugation kann somit als Sedimentation unter der Wirkung einer künstlich erhöhten Fallbeschleunigung aufgefaßt werden. Die sich bewegenden Teilchen werden durch das elektrische Feld des ersten Paares der Mikroelektroden größenabhängig getrennt.

Die Darstellung in Fig. 15 zeigt die Verhältnisse während der Sedimentation bzw. Zentrifugation. Durch die exakt einstellbaren Zentrifugalkräfte über die Rotationsgeschwindigkeit bewegen sich die Teilchen in den unteren Teil des Mikrosystems. Entsprechend der üblichen Zentrifugationsprinzipien sedimentieren die Teilchen mit der größten Dichte zuerst. Da die Teilchen 21 durch die elektrische Feldbarriere im Kanal nach rechts verschoben werden, während die Teilchen 22 davon unbeeinflußt bleiben, so ergibt sich in den Kanalenden 29a, 29b eine Trennung beider Teilchenarten. Die Teilchen in jedem der Kanalenden ordnen sich zusätzlich wie bei der üblichen Zentrifugation entsprechend ihrer Dichte an. Das dargestellte Mikrosystem kann als Grundform einer Vorrichtung betrachtet werden, wobei diese Grundform anwendungsabhängig vergrößert, erweitert oder mit weiteren Mikrostrukturen kombiniert werden kann. Der Vorteil besteht darin, daß keine Lösungsströmung entsteht und dennoch die Partikelbewegung gerichtet und einstellbar ist. Derartige Systeme können auch entgegengesetzte Bewegungen erzeugen, wenn die Teilchen einen Auftrieb besitzen.

Ausgehend von der dargestellten Grundform kann ein Mikrosystem beliebig erweitert werden, wie es an sich von den dielektrophoretischen Mikrosystemen bekannt ist. Demnach kann die Kanalstruktur insbesondere mehrere, über Verzweigungen miteinander verbundene Einzelkanäle aufweisen. Die Kanäle können gerade oder gekrümmt sein. Gekrümmte Kanalformen (z.B. Bögen, Mäander, Biegungen, Winkel usw.) können insbesondere zur Untersuchung von Bindungsunterschieden von Partikeln mit den Kanalwänden verwendet werden.

Gemäß einer weiteren Modifikation kann das Mikrosystem an der Aufnahme 12 (s. Fig. 14) drehbar angebracht sein. Während eines ersten Zentrifugationsvorganges erfolgt in einer ersten Mikrosystemorientierung z.B. eine Teilchentrennung gemäß Fig. 15.

Anschließend wird die Orientierung des Mikrosystems um 180° verändert, so daß die Gravitations- und/oder Zentrifugalkräfte entgegengesetzt der Pfeilrichtung 23 wirken. Die Kanalenden 29a, 29b übernehmen dann die Funktion von Eingangsdepots, von denen bei Vorhandensein geeigneter Kanalstrukturen (zusätzliche seitliche Abzweigungen) eine weitere Verteilung der getrennten Teilchen in Untergruppen oder eine bestimmte Behandlung (Beladen mit Stoffen, Elektroporation u. dgl.) erfolgen kann. Es sind auch in Abhängigkeit von der Kanalstruktur andere Orientierungsänderungen als die genannte 180°-Umkehr möglich. Es besteht ferner die Möglichkeit, die Aufnahme 12 so zu gestalten, daß das Mikrosystem während der Zentrifugation gedreht wird.

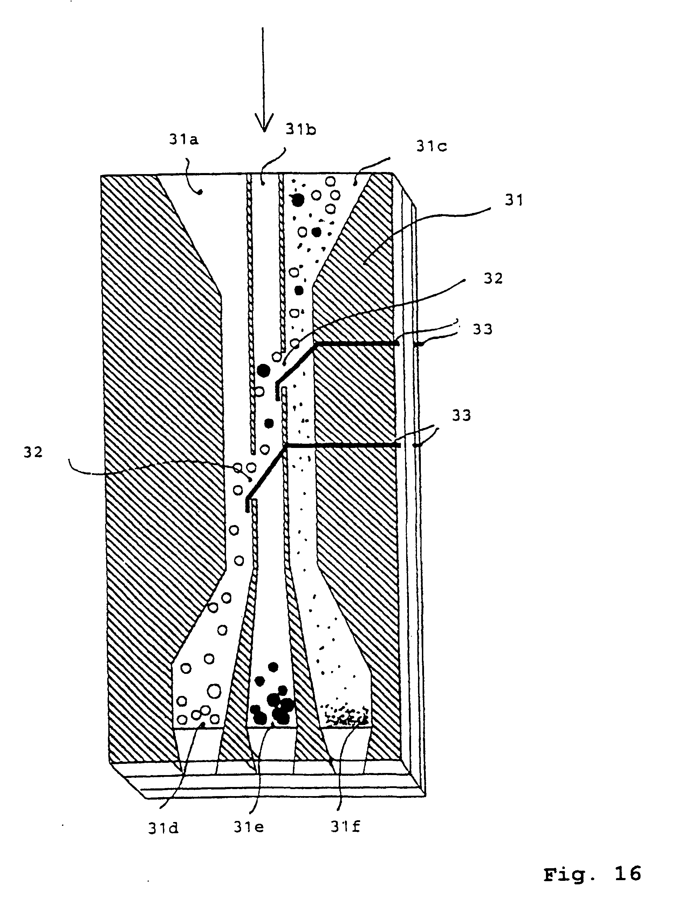

Ein programmierbares Beladungsmikrosystem für Zellen oder Teilchen ist in Fig. 16 gezeigt. Hier ist der Zentrifugationskanal in drei Teile 31a, 31b, 31c unterteilt. In den Zwischenwänden befinden sich Öffnungen 32, durch die wieder Elektroden 33 auf der Ober- und Unterseite des Kanals hindurchreichen. Die Öffnungen sind der Teilchengröße angepaßt (typischerweise 5- bis 20-fach größer als der Durchmesser). Zu Beginn werden in jeden der Kanalteile 31a bis 31c verschiedene Lösungen eingefüllt, die der chemischen Veränderung oder Beladung der Partikel dienen. Danach werden in einen Kanalteil (hier z.B. 31c) die Teilchen eingefügt. Durch die Zentrifugation gelangen die Teilchen (z.B. zuerst die schwarzen, dann die hellen) an die Elektroden 33 und können so automatisch über die elektrischen Feldbarrieren durch die Öffnungen 32 in die Nachbarlösungen überführt werden.

Auch hier kommt es zu einer Sortierung in den drei Kanalenden 31d, 31e, 31f und gleichzeitig zu einer Anordnung der Teilchen entsprechend der Masseunterschiede.

Weitere Eigenschaften der Mikrosysteme bestehen darin, daß sie Öffnungen (Zuflüsse, Durchflüsse, Abflüsse) besitzen können, die sich verschließen lassen, so daß die Teilchen nach der Zentrifugation oder davor leicht entnommen oder eingefügt werden können. Ferner können all die Mikroelektrodenelemente (Halteeiektroden für Teilchen, Mikrofeldkäfige etc.) eingebaut werden, die für die dielektrophoretische Beeinflussung von Teilchen an sich bekannt sind und bei herkömmlichen Mikrosystemen, die mit strömenden Flüssigkeiten arbeiten, eingesetzt werden. Aufgrund des Zusammenwirkens der Gravitations- bzw. Zentrifugalkräfte mit den dielekrophoretischen Kräften ist das Verfahren eine elektrisch gesteuerte oder aktive Zentrifugation. Zusätzlich können Kombinationen mit der Einwirkung optischer Kräfte (Laser-Tweezer), magnetischer Kräfte (Einwirkung auf magnetische Partikel) oder mechanischer Kräfte in Form von Ultraschallkräften vorgesehen sein.

Anwendungsgebiete sind insbesondere:
Zelltrennung/-fraktionierung, Zellsortierung, Zellbeladung (molekular, Nanoteilchen, Beads), Zellentladung (molekular), Zellpermeation (sog. Elektroporation), Zellfusion (sog. Elektrofusion), Zellpärchenbildung, und Zellaggregatbildung.

Das Verfahren ist nicht auf bestimmte Lösungs- oder Suspensionsflüssigkeiten beschränkt. Es ist vorteilhaft, wenn die Viskosität der im Mikrosystem enthaltenen Flüssigkeit bekannt ist. Bei bekannter Viskosität läßt sich die Drehzahl zur Einstellung einer bestimmten Partikelgeschwindigkeit auf der Grundlage von Tabellenwerten oder durch einen Programmalgorithmus ermitteln. Alternativ ist es jedoch auch möglich, die tatsächliche Geschwindigkeit der Partikel im Mikrosystem während der Zentrifugation zu erfassen (z.B. mit einem optischen Sensor) und die Drehzahl zur Einstellung einer bestimmten Partikelgeschwindigkeit zu regeln. Es kann vorgesehen sein, daß in verschiedenen Teilbereichen des Kanalstrukturen, z.B. in parallel verlaufenen Kanälen, die nur über eine Öffnung miteinander verbunden sind, Flüssigkeiten mit verschiedenen Viskositäten enthalten sind. In diesem Fall werden jedoch Viskositäten bevorzugt, bei denen sichergestellt ist, daß die Diffusion der Flüssigkeiten durch die Öffnung über den Zentrifugationszeitraum verhältnismäßig klein oder vernachlässigbar klein ist.

Falls die Massendichte der Partikel kleiner als die Flüssigkeit im Mikrosystem ist, kann das Verfahren entsprechend abgewandelt implementiert werden, indem Partikel gegebenenfalls auf der der Drehachse abgewandten Seite des Mikrosystems eingebracht werden und unter Wirkung des Auftriebs oder unter kombinierter Wirkung des Auftriebs und der Zentrifugalkräfte zum anderen Ende des Mikrosystems wandern.

Das Mikrosystem wird anwendungsabhängig in Bezug auf die Kanalstruktur und die Ausrichtung der Elektrodeneinrichtungen angepaßt. Die Kanalquerdimensionen sind in der Regel wesentlich größer als die Durchmesser der einzelnen Partikel. Dadurch wird vorteilhafterweise ein Verstopfen der Kanäle vermieden. Sind lediglich Partikel mit besonders geringen Dimensionen zu manipulieren (z.B. Bakterien oder Viren oder Zellorganellen), so können die Kanaldimensionen entsprechend verringert werden, z.B. auf Beträge unterhalb 10 µm.

Das Verfahren wird mit einem Mikrosystem implementiert, das mindestens einseitig geschlossen ist. Das geschlossene Ende kann ein geschlossenes Kanalende, eine geschlossene Sammelzohe oder auch ein geschlossener Hohlraum im Mikrosystem sein. Bei der Partikelmanipulation erfolgt im wesentlichen keine Flüssigkeitsbewegung hin zu dem geschlossenen Ende. Dies bedeutet, insbesondere bei Realisierung von Sammelzonen oder Hohlräumen am geschlossenen Ende, daß diese wie das gesamte Mikrosystem zu Beginn der Partikelmanipulation mit der Lösung oder Suspension für die Teilchen gefüllt ist.

Falls es beim Manipulieren der Partikel zu Zusammenballungen oder vorübergehenden Verstopfungen der Kanalstrukturen kommt, so ist vorgesehen, die Drehzahl der Zentrifuge kurzzeitig zu erhöhen, um so die zusammenhaftenden Partikel abzulösen und weiter zu bewegen.

## Patentansprüche

1. Mikrosystem (20), das zur dielektrophoretischen Manipulierung von Teilchen (30, 30a, 30b) in einer Suspensionsflüssigkeit in einem Kanal (21, 211, 212) eingerichtet ist und eine Elektrodenanordnung (10) mit mindestens einer Mikroelekrode (11, 11a-e, 12, 41a-f, 47, 51a, 51b, 511b, 512b,61a-e, 612a, 71a, 71b, 711a) auf einer seitlichen Wand (21a, 21b, 23) des Kanals zur Erzeugung einer Feldbarriere enthält, die den Kanal zumindest teilweise durchsetzt,
**dadurch gekennzeichnet, dass**
die Mikroelektrode in Bezug auf die Strömungsrichtung im Kanal einen vorbestimmten stetigen Krümmungsverlauf besitzt oder aus einer Vielzahl jeweils gerader Elektrodenabschnitte mit vorbestimmten Winkeln in Bezug auf die Strömungsrichtung besteht, so dass die Feldbarriere einen vorbestimmten Krümmungsverlauf relativ zur Strömungsrichtung besitzt.

2. Mikrosystem gemäß Anspruch 1, in dem die Elektrodenanordnung mindestens zwei an gegenüberliegenden Kanalwänden angebrachte Mikroelektroden (11, 12) gleicher Gestalt und Ausrichtung umfasst, die jeweils die Form eines gekrümmten Bandes besitzen.

3. Mikrosystem gemäß Anspruch 2, in dem die Mikroelektroden (11a-e, 41a-f) in Abhängigkeit vom Strömungsprofil so gekrümmt sind, dass in jedem Abschnitt der Feldbarriere der Mikroelektrode die auf ein Teilchen wirkende, aus Polarisations- und Strömungskräften resultierende Kraft in einen Bereich weist, der in Bezug auf die Strömungsrichtung vor der Mikroelektrode gelegen ist.

4. Mikrosystem gemäß Anspruch 3, in dem vier Mikroelektroden als Fokussierelektroden (11a-e) zur Bildung eines Partikeltrichters angeordnet sind.

5. Mikrosystem gemäß Anspruch 2, in dem die Mikroelektroden (11a-e, 41a-f) in Abhängigkeit vom Strömungsprofil so gekrümmt sind, dass die auf ein Teilchen wirkende, aus Polarisationsund Strömungskräften resultierende Kraft von einem relativ zur Strömungsrichtung ersten Ende der Mikroelektrode hin zum anderen Ende eine Richtungsänderung durchläuft, die von einer Richtung in einen Bereich in Bezug auf die Strömungsrichtung vor der Mikroelektrode zu einer Richtung in einen Bereich in Bezug auf die Strömungsrichtung nach der Mikroelektrode führt.

6. Mikrosystem gemäß Anspruch 5, in dem zwei Mikroelektroden als Sortierelektroden (11a-e, 41a-f) vorgesehen sind, deren Feldbarriere mit dem Strömungsprofil der Suspensionsflüssigkeit im Kanal so zusammenwirkt, dass suspendierte Teilchen mit verschiedenen passiven elektrischen Eigenschaften die Sortierelektroden je nach ihren Eigenschaften auf getrennten Bahnen passieren können.

7. Mikrosystem gemäß Anspruch 2, in dem an gegenüberliegenden Kanalwänden mindestens zwei Mikroelektroden (61a-e, 611a-e, 612a, 71a, 71b, 711a) gleicher Gestalt und Ausrichtung vorgesehen sind, die jeweils einen in Strömungsrichtung geschlossenen Winkelabschnitt aufweisen.

8. Mikrosystem gemäß Anspruch 7, in dem die Mikroelektroden als Fangelektroden (61a-e, 611a-e, 612a, 71a, 71b, 711a) zusammenwirken.

9. Mikrosystem gemäß Anspruch 7 oder 8, in dem eine Gruppe von Fangelektroden (61a-e, 611a-e, 612a, 71a, 71b, 711a) in Kanalquerrichtung angeordnet sind.

10. Mikrosystem gemäß einem der vorhergehenden Ansprüche, in dem die Mikroelektroden paarweise jeweils auf den Boden- und Deckflächen des Kanals angeordnet sind.

11. Mikrosystem gemäß Anspruch 1, in dem drei Mikroelektroden vorgesehen sind, von denen zwei Mikroelektroden als Fokussierelektroden (41a-d) in Form von zu einer Mittellinie konvergierenden, bandförmigen Elektroden an den Boden- und Deckflächen des Kanals angebracht und eine dritte Mikroelektrode als feldformende Hilfselektrode (47) mit Abstand von den Boden- und Deckflächen in der Mitte des Kanals angeordnet ist.

12. Mikrosystem gemäß Anspruch 11, in dem der Kanal (211, 212) durch eine Trennwand (231) in zwei Teilkanäle mit einer Öffnung stromaufwärts in Bezug auf die Hilfselektrode (103) geteilt ist.

13. Verwendung eines Mikrosystems gemäß einem der Ansprüche 1 bis 12 zum Ablenken, Sortieren, Sammeln und/oder Formieren von mikroskopisch kleinen Teilchen.

## Claims

1. A microsystem (20) which is arranged for dielectrophoretic manipulation of particles (30, 30a, 30b) in a suspension fluid in a channel (21, 211, 212) and comprises an electrode arrangement (10) with at least one micro-electrode (11, 11a-e, 12, 41a-f, 47, 51a, 51b, 511b, 512b, 61a-e, 612a, 71a, 71b, 711a) on a sidewall (21a, 21b, 23) of the channel for creating a field barrier which passes at least partially through the channel, **characterized in that** the micro-electrode has a predetermined continuously curved course in relation to the flow direction in the channel or consists of a plurality of straight electrode sections with predetermined angles relative to the flow direction, so that the field barrier has a predetermined curved course relative to the flow direction.

2. A microsystem according to claim 1, in which the electrode arrangement comprises at least two micro-electrodes (11, 12) of like form and alignment applied to opposite channel walls, each having the form of a curved band.

3. A microsystem according to claim 2, in which the micro-electrodes (11a-e, 41a-f) are so curved in dependence on the flow profile that, in each section of the field barrier of the micro-electrode, the force acting on a particle, resulting from polarisation and flow forces, points into a region which is located in front of the micro-electrode in relation to the flow direction.

4. A microsystem according to claim 3, in which four micro-electrodes are arranged as focusing electrodes (11a-e) to form a particle funnel.

5. A microsystem according to claim 2, in which the micro-electrodes (11a-e, 41a-f) are so curved in dependence on the flow profile that the force acting on a particle, resulting from polarisation and flow forces, passes through a change of direction from a first end relative to the flow direction of the micro-electrode to the other end, which leads from a direction into a region relative to the flow direction in front of the micro-electrode to a direction into a region relative to the flow direction after the micro-electrode.

6. A microsystem according to claim 5, in which two micro-electrodes are provided as sorting electrodes (11a-e, 41a-f), whose field barrier so cooperates with the flow profile of the suspension fluid in the channel that suspended particles with different passive electrical properties can pass the sorting electrodes on separate paths in accordance with their properties.

7. A microsystem according to claim 2, in the at least two micro-electrodes (61a-e, 611a-e, 612a, 71a, 71b, 711a) of like form and alignment are provided on opposite channel walls, each having an angle section closed in the flow direction.

8. A microsystem according to claim 7, in which the micro-electrodes cooperate as trapping electrodes (61a-e, 611a-e, 612a, 71a, 71b, 711a).

9. A microsystem according to claim 7 or 8, in which a group of trapping electrodes (61a-e, 611a-e, 612a, 71a, 71b, 711a) are arranged in the channel transverse direction.

10. A microsystem according to any of the preceding claims, in which the micro-electrodes are arranged in pairs on the bottom and top surfaces of the channel.

11. A microsystem according to claim 1, in which three micro-electrodes arc provided, of which two micro-electrodes are applied to the bottom and top surfaces of the channel as focusing electrodes (41a-d) in the form of band shaped electrodes converging to a centre line and.a third micro-electrode is arranged in the middle of the channel, spaced from the bottom and top surfaces, as a field-forming auxiliary electrode (47).

12. A microsystem according to claim 11, in which the channel (211, 212) is divided by a partition (231) into two sub-channels with an opening upstream in relation to the auxiliary electrode (103).

13. Use of a microsystem according to any of claims 1 to 12 for deflecting, sorting, collecting and/or formation of microscopic small particles.

## Revendications

1. Microsystème (20) équipé pour une manipulation diélectrophorétique de particules (30, 30a, 30b) dans un liquide de suspension dans un canal (21, 211, 212) et comprenant un ensemble d'électrodes (10) avec au moins une microélectrode (11, 11a-e, 12, 41a-f, 47, 51a, 51b, 511b, 512b, 61a-e, 612a, 71a, 71b, 711a) sur une paroi latérale (21a, 21b, 23) du canal pour la production d'une barrière de champ qui traverse au moins partiellement le canal, **caractérisé en ce que** la microélectrode suit par rapport à la direction d'écoulement dans le canal un cheminement en courbe continu prédéterminé ou se compose d'une pluralité de sections d'électrodes respectivement droites avec des angles prédéterminés en fonction de la direction de l'écoulement, de sorte que la barrière de champ suit par rapport à la direction de l'écoulement un cheminement en courbe prédéterminé.

2. Microsystème selon la revendication 1, **caractérisé en ce que** l'ensemble d'électrodes comprend au moins deux microélectrodes (11, 12) de même configuration et de même orientation, qui sont disposées sur des parois de canaux opposées et qui ont respectivement la forme d'une bande courbe.

3. Microsystème selon la revendication 2, **caractérisé en ce que** les microélectrodes (11a-e, 41a-f) sont recourbées de telle manière en fonction du profil d'écoulement que, dans chaque section de la barrière de champ de la microélectrode, la force agissant sur une particule et résultant d'efforts de polarisation et d'écoulement est dirigée vers une zone qui, par rapport à la direction d'écoulement, est placée devant la microélectrode.

4. Microsystème selon la revendication 3, **caractérisé en ce que** quatre microélectrodes sont disposées en tant qu'électrodes de focalisation (11a-e) pour la formation d'un entonnoir à particules.

5. Microsystème selon la revendication 2, **caractérisé en ce que** les microélectrodes (11a-e, 41a-f) sont recourbées de telle manière en fonction du profil d'écoulement que la force agissant sur une particule et résultant d'efforts de polarisation et d'écoulement effectue, depuis une extrémité de la microélectrode se trouvant en premier lieu par rapport à la direction d'écoulement jusqu'à l'autre extrémité, un changement de direction qui conduit depuis une direction dans une zone se rapportant à la direction d'écoulement se trouvant devant la microélectrode vers une direction dans une zone se rapportant à la direction d'écoulement se trouvant derrière la microélectrode.

6. Microsystème selon la revendication 5, **caractérisé en ce que** deux microélectrodes sont prévues comme électrodes de triage (11a-e, 41a-f), dont la barrière de champ est en interaction telle avec le profil d'écoulement du liquide de suspension dans le canal que des particules suspendues avec diverses caractéristiques électriques passives peuvent, selon leurs caractéristiques, passer les électrodes de triage sur des voies séparées.

7. Microsystème selon la revendication 2, **caractérisé en ce que** sur des parois de canaux opposées sont prévues au moins deux microélectrodes (61a-e, 611a-e, 612a, 71a, 71b, 711a) de même configuration et de même orientation, qui comprennent respectivement une section d'angle fermée en direction d'écoulement.

8. Microsystème selon la revendication 7, **caractérisé en ce que** les microélectrodes agissent de concert en tant qu'électrodes collectrices (61a-e, 611a-e, 612a, 71a, 71b, 711a).

9. Microsystème selon la revendication 7 ou 8, **caractérisé en ce qu'**un groupe d'électrodes collectrices (61a-e, 611a-e, 612a, 71a, 71b, 711a) est disposé en direction transversale du canal.

10. Microsystème selon l'une quelconque des revendications précédentes, **caractérisé en ce que** les microélectrodes sont disposées par paires respectivement sur les surfaces du fond et de couverture du canal.

11. Microsystème selon la revendication 1, **caractérisé en ce que** trois microélectrodes sont prévues, dont deux microélectrodes sont disposées sur les surfaces du fond et de couverture du canal en tant qu'électrodes de focalisation (41a-d) sous la forme d'électrodes en forme de bandes convergeant vers une ligne médiane et une troisième microélectrode est disposée avec un espacement par rapport -aux surfaces du fond et de couverture au centre du canal en tant qu'électrode auxiliaire (47) formant un champ.

12. Microsystème selon la revendication 11, **caractérisé en ce que** le canal (211, 212) est divisé par une paroi de séparation (231) en deux canaux partiels avec une ouverture se trouvant en amont de l'écoulement par rapport à l'électrode auxiliaire (103).

13. Utilisation d'un microsystème selon l'une quelconque des revendications 1 à 12 pour la déviation, le triage, la collecte et/ou la formation de particules microscopiques.
